# EUROPEAN PATENT APPLICATION

(11) **EP 4 335 459 A1**
(43) Date of publication of application: **13.03.2024**
(21) Application number: 22807516.4
(22) Date of filing: 11.05.2022
(51) Int. Cl.: A61K 47/68, A61K 9/08, A61K 9/19, A61K 38/46, A61K 39/395, A61K 47/02, A61K 47/04, A61K 47/10, A61K 47/12, A61K 47/18, A61K 47/26, A61P 3/00, A61P 25/00, C12N 15/13, C12N 15/56, C12N 15/62

(54) **PHARMACEUTICAL COMPOSITION FOR TREATMENT OF MUCOPOLYSACCHARIDOSIS TYPE 1**

(30) Priority: 12.05.2021 JP 2021080729
(71) Applicant: JCR Pharmaceuticals Co., Ltd., Ashiya-shi, Hyogo 659-0021 (JP)
(72) Inventor: MORIMOTO, Hideto, Kobe-shi, Hyogo 651-2241 (JP); KIDA, Sachiho, Ashiya-shi, Hyogo 659-0021 (JP); SOU, Sairei, Ashiya-shi, Hyogo 659-0021 (JP); KAWASHIMA, Satoshi, Ashiya-shi, Hyogo 659-0021 (JP)
(74) Representative: Boult Wade Tennant LLP
(86) International application number: PCT/JP2022/020001
(87) International publication number: WO 2022/239817

(57) **Abstract**

Provided is a pharmaceutical composition for treatment of mucopolysaccharidosis type I patients. Here, the patient has a disorder particularly in the central nervous system. A pharmaceutical composition containing, as an active ingredient, a fusion protein of an anti-human transferrin receptor antibody and human α-L-iduronidase is administered to a patient with mucopolysaccharidosis type I by intravenous infusion at a dose of 0.1 to 10 mg/kg body weight. The administration is performed continually for at least 3 months at intervals of 5 days to 21 days. This decomposes dermatan sulfate and heparan sulfate accumulated in the tissues of the patient, particularly in the central nervous system.

## Description

### Technical Field

The present invention relates to a pharmaceutical composition for treatment of mucopolysaccharidosis type I, the composition containing, as an active ingredient, a fusion protein of an anti-human transferrin receptor antibody and human α-L-iduronidase (hIDUA), and specifically relates to a pharmaceutical composition for treatment of mucopolysaccharidosis type I, the composition characterized by decomposing dermatan sulfate and heparan sulfate accumulated in organs including the brain by parenteral administration of the pharmaceutical composition to a patient with mucopolysaccharidosis type I.

### Background Art

α-L-iduronidase (IDUA) is one of lysosomal enzymes having an activity of hydrolyzing non-sulfated α-L-iduronosidic linkages present in molecules of glycosaminoglycans (GAGs), such as heparan sulfate and dermatan sulfate. Patients with mucopolysaccharidosis type I are genetically partially or completely deficient in α-L-iduronidase activity. A deficiency in this enzyme leads to abnormal metabolism of heparan sulfate and dermatan sulfate, and that in turn leads to accumulation of fragments of those molecules in tissues such as the liver and kidney, and further excretion of heparan sulfate and dermatan sulfate in urine. Consequently, these abnormalities cause a variety of symptoms in patients with mucopolysaccharidosis type I, including skeletal deformities and severe mental retardation. Mucopolysaccharidosis type I is classified into severe Hurler syndrome (MPS IH), intermediate Hurler-Scheie syndrome (MPS IH-S), and mild Scheie syndrome (MPS IS).

The fact that patients with mucopolysaccharidosis type I show only slight IDUA activity was already known in the 1970s, and it has been hypothesized that an abnormality in the IDUA gene is the cause of this disease. In 1991, a human gene encoding hIDUA was isolated and identified as a causative gene for this disease (Non-Patent Literature 1). The isolation of the gene encoding hIDUA has made it possible to mass-produce recombinant hIDUA (rhIDUA) using recombinant technology and use this enzyme as a therapeutic agent in enzyme replacement therapy for mucopolysaccharidosis type I (Patent Literature 1).

However, rhIDUA cannot cross the blood-brain barrier (BBB) and thus cannot effectively alleviate a disorder of the central nervous system in mucopolysaccharidosis type I patients. To solve such an issue for enzyme replacement therapy for mucopolysaccharidosis type I using rhIDUA, a fusion protein of an anti-human transferrin receptor antibody and human α-L-iduronidase has been developed (Patent Literatures 2 and 3). This fusion protein can cross the BBB when the anti-human transferrin receptor antibody of the fusion protein binds to the human transferrin receptor present on capillary endothelial cells in the brain constituting the BBB.

### Citation List

### Patent Literature

Patent Literature 1: US 6149909
Patent Literature 2: US 2018/0171012
Patent Literature 3: US 2019/0338043

### Non-Patent Literature

Non-Patent Literature 1: Scott HS. et. al., Proc Natl Acad Sci USA 88: 9695-9 (1991)

### Summary of Invention

### Technical Problem

An object of the present invention is to provide a method for treating a patient with mucopolysaccharidosis type I, characterized by decomposing glucosaminoglycans accumulated at least in the brain by parenteral administration of a pharmaceutical composition containing, as an active ingredient, a fusion protein of an anti-human transferrin receptor antibody and human α-L-iduronidase to a patient with mucopolysaccharidosis type I.

### Solution to Problem

In a study aimed at the above object, the present inventors have found that glucosaminoglycans accumulated in the brain of a patient with mucopolysaccharidosis type I can be decomposed by intravenous injection of a pharmaceutical composition containing, as an active ingredient, a fusion protein of an anti-human transferrin receptor antibody and human α-L-iduronidase (hIDUA) to a patient with mucopolysaccharidosis type I and completed the present invention. That is, the present invention includes the following.
1. A pharmaceutical composition containing, as an active ingredient, a fusion protein of an anti-human transferrin receptor antibody and human α-L-iduronidase, in which the fusion protein is administered to a patient with mucopolysaccharidosis type I by intravenous infusion at a dose of 0.1 to 10 mg/kg body weight.
2. The pharmaceutical composition according to 1 above, in which the fusion protein is administered at a dose of 0.1 to 8 mg/kg body weight.
3. The pharmaceutical composition according to 1 above, in which the fusion protein is administered at a dose of 1 to 6 mg/kg body weight.
4. The pharmaceutical composition according to 1 above, in which the fusion protein is administered at a dose of 2 mg/kg body weight or 4 mg/kg body weight.
5. The pharmaceutical composition according to any of 1 to 4 above, in which the fusion protein is administered at a rate of 0.33 mg/hour to 200 mg/hour.
6. The pharmaceutical composition according to any of 1 to 4 above, in which the fusion protein is administered over at least 1 hour.
7. The pharmaceutical composition according to any of 1 to 6 above, in which the fusion protein is administered by intravenous drip.
8. The pharmaceutical composition according to any of 1 to 7 above, in which the administration is performed continually for at least 3 months at intervals of 5 days to 21 days.
9. The pharmaceutical composition according to any of 1 to 7 above, in which the fusion protein is administered continually for at least 1 month at intervals of 7 days.
10. The pharmaceutical composition according to 8 or 9 above, in which the fusion protein is administered at a dose of 0.1 to 2 mg/kg body weight in a first administration and is administered at an increased dose in second and subsequent administrations.
11. The pharmaceutical composition according to 8 or 9 above, in which the fusion protein is administered at a dose of 0.1 to 2 mg/kg body weight in a first administration and is then administered at a maintenance dose of 2 to 6 mg/kg body weight.
12. The pharmaceutical composition according to 8 or 10 above, the fusion protein is administered at a maintenance dose of 2 mg/kg body weight or 4 mg/kg body weight.
13. The pharmaceutical composition according to any of 1 to 12 above, in which the anti-human transferrin receptor antibody is a Fab.
14. The pharmaceutical composition according to any of claims 1 to 13, in which the human α-L-iduronidase is linked directly or via a linker to a light chain of the anti-human transferrin receptor antibody at the C-terminal side or the N-terminal side, or to a heavy chain of the anti-human transferrin receptor antibody at the C-terminal side or the N-terminal side.
15. The pharmaceutical composition according to any of 1 to 13 above, in which the human α-L-iduronidase is linked via a linker to the heavy chain of the anti-human transferrin receptor antibody at the C-terminal side.
16. The pharmaceutical composition according to 14 or 15 above, in which the linker is a peptide consisting of 1 to 150 amino acid residues.
17. The pharmaceutical composition according to 16 above, in which the linker is a peptide including the amino acid sequence selected from the group consisting of one glycine, one serine, the amino acid sequence Gly-Ser, the amino acid sequence Gly-Gly-Ser, the amino acid sequence of SEQ ID NO: 1, the amino acid sequence of SEQ ID NO: 2, the amino acid sequence of SEQ ID NO: 3, and the amino acid sequence consisting of 1 to 10 of the aforementioned amino acid sequences that are consecutively linked.
18. The pharmaceutical composition according to 16 above, in which the linker is a peptide including the amino acid sequence of SEQ ID NO: 3.
19. The pharmaceutical composition according to any of 1 to 18 above, in which
   the anti-human transferrin receptor antibody includes, in a variable region of the light chain, the amino acid sequence of SEQ ID NO: 4 or SEQ ID NO: 5 as CDR1, the amino acid sequence of SEQ ID NO: 6 or SEQ ID NO: 7 or the amino acid sequence Lys-Val-Ser as CDR2, and the amino acid sequence of SEQ ID NO: 8 as CDR3, and includes, in a variable region of the heavy chain, the amino acid sequence of SEQ ID NO: 9 or 10 as CDR1, the amino acid sequence of SEQ ID NO: 11 or 2 as CDR2, and the amino acid sequence of SEQ ID NO: 13 or 14 as CDR3, and
   the human α-L-iduronidase is linked to the light chain of the anti-human transferrin receptor antibody at the C-terminal side or the N-terminal side, or to the heavy chain of the anti-human transferrin receptor antibody at the C-terminal side or the N-terminal side.
20. The pharmaceutical composition according to 19 above, in which the variable region of the heavy chain includes the amino acid sequence of SEQ ID NO: 16.
21. The pharmaceutical composition according to 20 above, in which the heavy chain is a Fab heavy chain, and the Fab heavy chain includes the amino acid sequence of SEQ ID NO: 19.
22. The pharmaceutical composition according to any of 19 to 21, in which the variable region of the light chain includes the amino acid sequence of SEQ ID NO: 17.
23. The pharmaceutical composition according to 22 above, in which the light chain includes the amino acid sequence of SEQ ID NO: 18.
24. The pharmaceutical composition according to any of 1 to 23 above, in which the human α-L-iduronidase includes the amino acid sequence having at least 85% identity with the amino acid sequence of SEQ ID NO: 20 or the amino acid sequence of SEQ ID NO: 21.
25. The pharmaceutical composition according to any of 1 to 23 above, in which the human α-L-iduronidase includes the amino acid sequence of SEQ ID NO: 20 or the amino acid sequence of SEQ ID NO: 21.
26. The pharmaceutical composition according to 1 above, in which
   the light chain of the anti-human transferrin receptor antibody includes the amino acid sequence of SEQ ID NO: 18,
   the heavy chain of the anti-human transferrin receptor antibody includes the amino acid sequence of SEQ ID NO: 19, and
   the heavy chain is linked at the C-terminal side of the heavy chain to the human α-L-iduronidase with the amino acid sequence of SEQ ID NO: 20 or SEQ ID NO: 21 via the linker of the amino acid sequence of SEQ ID NO: 3.
27. The pharmaceutical composition according to 19 above, in which
   the light chain of the anti-human transferrin receptor antibody includes the amino acid sequence of SEQ ID NO: 18,
   the heavy chain of the anti-human transferrin receptor antibody includes the amino acid sequence of SEQ ID NO: 19, and
   the heavy chain is linked at the C-terminal side of the heavy chain to the human α-L-iduronidase with the amino acid sequence of SEQ ID NO: 20 via the linker of the amino acid sequence of SEQ ID NO: 3, thereby to form an amino acid sequence of SEQ ID NO: 24.
28. The pharmaceutical composition according to any of 1 to 27 above, in which the pharmaceutical composition is a lyophilized preparation or an aqueous liquid preparation.
29. The pharmaceutical composition according to 28 above, further containing at least one selected from the group consisting of a neutral salt, a disaccharide, a nonionic surfactant, and a buffer.
30. The pharmaceutical composition according to 28 or 29 above, in which the pharmaceutical composition contains a polysorbate and/or a poloxamer as the nonionic surfactant.
31. The pharmaceutical composition according to 30 above, in which
   the polysorbate is polysorbate 20 or polysorbate 80, and
   the poloxamer is selected from the group consisting of poly(oxyethylene) (54) poly(oxypropylene) (39) glycol, poly(oxyethylene) (196) poly(oxypropylene) (67) glycol, poly(oxyethylene) (42) poly(oxypropylene) (67) glycol, poly(oxyethylene) (3) poly(oxypropylene) (17) glycol, poly(oxyethylene) (20) poly(oxypropylene) (20) glycol, and poly(oxyethylene) (120) poly(oxypropylene) (40) glycol.
32. The pharmaceutical composition according to 30 above, in which
   the polysorbate is polysorbate 80, and
   the poloxamer is poly(oxyethylene) (160) poly(oxypropylene) (30) glycol.
33. The pharmaceutical composition according to any of 30 to 32 above, in which the pharmaceutical composition is an aqueous liquid preparation,
   a concentration of the polysorbate is from 0.005 to 1.5 mg/mL, and
   a concentration of the poloxamer is from 0.1 to 0.6 mg/mL.
34. The pharmaceutical composition according to any of 30 to 32 above, in which the pharmaceutical composition is an aqueous liquid preparation,
   a concentration of the polysorbate is from 0.025 to 1.0 mg/mL, and
   a concentration of the poloxamer is from 0.2 to 0.5 mg/mL.
35. The pharmaceutical composition according to any of 30 to 32 above, in which the pharmaceutical composition is an aqueous liquid preparation,
   a concentration of the polysorbate is from 0.05 to 0.15 mg/mL, and
   a concentration of the poloxamer is from 0.25 to 0.45 mg/mL.
36. The pharmaceutical composition according to any of 29 to 35 above, in which the neutral salt is sodium chloride.
37. The pharmaceutical composition according to any of 29 to 36 above, in which the disaccharide is selected from the group consisting of trehalose, sucrose, maltose, lactose, and a combination of two or more thereof.
38. The pharmaceutical composition according to any of 29 to 37 above, in which the buffer is selected from the group consisting of a citrate buffer, a phosphate buffer, a glycine buffer, a histidine buffer, a carbonate buffer, an acetate buffer, and a combination of two or more thereof.
39. The pharmaceutical composition according to 30 to 32 above, in which the pharmaceutical composition is an aqueous liquid preparation selected from the group consisting of:
   (1) an aqueous liquid preparation with a concentration of the fusion protein of 1 to 10 mg/mL, a concentration of the neutral salt of 0.3 to 1.2 mg/mL, a concentration of the disaccharide of 50 to 100 mg/mL, a concentration of the buffer of 10 to 30 mM, a concentration of the polysorbate of 0.005 to 1.5 mg/mL, and a concentration of the poloxamer of 0.1 to 0.6 mg/mL;
   (2) an aqueous liquid preparation with a concentration of the fusion protein of 2 to 8 mg/mL, a concentration of the neutral salt of 0.5 to 1.0 mg/mL, a concentration of the disaccharide of 55 to 95 mg/mL, a concentration of the buffer of 15 to 25 mM, a concentration of the polysorbate of 0.05 to 1.0 mg/mL, and a concentration of the poloxamer of 0.25 to 0.45 mg/mL; and
   (3) an aqueous liquid preparation with a concentration of the fusion protein of 4 to 6 mg/mL, a concentration of the neutral salt of 0.7 to 0.9 mg/mL, a concentration of the disaccharide of 60 to 90 mg/mL, a concentration of the buffer of 15 to 25 mM, a concentration of the polysorbate of 0.05 to 0.15 mg/mL, and a concentration of the poloxamer of 0.25 to 0.45 mg/mL.
40. The pharmaceutical composition according to any of 28 to 41 above, in which the pharmaceutical composition is an aqueous liquid preparation with a pH of 4.5 to 6.5, 5.0 to 6.0, or 5.2 to 5.8.
41. The pharmaceutical composition according to any of 30 to 32 above, in which the pharmaceutical composition is a lyophilized preparation selected from the group consisting of:
   (1) a lyophilized preparation giving a concentration of the fusion protein of 1 to 10 mg/mL, a concentration of the neutral salt of 0.3 to 1.2 mg/mL, a concentration of the disaccharide of 50 to 100 mg/mL, a concentration of the buffer of 10 to 30 mM, a concentration of the polysorbate of 0.005 to 1.5 mg/mL, and a concentration of the poloxamer of 0.1 to 0.6 mg/mL when dissolved in pure water;
   (2) a lyophilized preparation giving a concentration of the fusion protein of 2 to 8 mg/mL, a concentration of the neutral salt of 0.5 to 1.0 mg/mL, a concentration of the disaccharide of 55 to 95 mg/mL, a concentration of the buffer of 15 to 25 mM, a concentration of the polysorbate of 0.05 to 1.0 mg/mL, and a concentration of the poloxamer of 0.25 to 0.45 mg/mL when dissolved in pure water; and
   (3) a lyophilized preparation giving a concentration of the fusion protein of 4 to 6 mg/mL, a concentration of the neutral salt of 0.7 to 0.9 mg/mL, a concentration of the disaccharide of 60 to 90 mg/mL, a concentration of the buffer of 15 to 25 mM, a concentration of the polysorbate of 0.05 to 0.15 mg/mL, and a concentration of the poloxamer of 0.25 to 0.45 mg/mL when dissolved in pure water.
42. The pharmaceutical composition according to any of 28 to 32, 36 to 38, and 41 above, in which the pharmaceutical composition is a lyophilized preparation giving a pH of 4.5 to 6.5, 5.0 to 6.0, or 5.2 to 5.8 when dissolved in pure water.
43. The pharmaceutical composition according to any of 1 to 42 above, in which the patient has a disorder in the central nervous system.
44. The pharmaceutical composition according to any of 1 to 43 above, in which the pharmaceutical composition has an effect of reducing concentrations of dermatan sulfate and heparan sulfate contained in cerebrospinal fluid, serum, and urine.
45. The pharmaceutical composition according to any of 1 to 44 above, in which the pharmaceutical composition is used in enzyme replacement therapy for a patient with mucopolysaccharidosis type I.
46. The pharmaceutical composition of 1 to 45 above, in which the pharmaceutical composition is used in combination with an immunosuppressive agent.
47. Enzyme replacement therapy for a patient with mucopolysaccharidosis type I, the enzyme replacement therapy including using the pharmaceutical composition described in any of 1 to 46 above.
48. The enzyme replacement therapy according to 47 above, in which the patient has a disorder in the central nervous system.
49. The pharmaceutical composition according to any of 1 to 46 above, in which when the pharmaceutical composition is administered to a patient with mucopolysaccharidosis type I, a concentration of heparan sulfate contained in cerebrospinal fluid collected from the patient after the administration is 2/3 or less, 1/2 or less, or 1/3 or less, compared with a concentration of heparan sulfate contained in cerebrospinal fluid collected from the patient before the first administration of the pharmaceutical composition.
50. The pharmaceutical composition according to any of 1 to 46 above, in which when the pharmaceutical composition is administered to a patient with mucopolysaccharidosis type I, a concentration of heparan sulfate contained in cerebrospinal fluid collected from the patient after the administration is 2000 ng/mL or less, 1800 ng/mL or less, 1600 ng/mL or less, 1500 ng/mL or less, or 1400 ng/mL or less.
50. The pharmaceutical composition according to any of 1 to 46 above, in which administration of the pharmaceutical composition to a patient with mucopolysaccharidosis type I leads to at least one of functional improvements described below in the patient compared to before the first administration of the pharmaceutical composition:
   (1) the patient is able to have a conversation for a longer period;
   (2) the patient is able to write more letters and/or characters;
   (3) joint pain in the lower back, knees, and the like is reduced;
   (4) pain and muscle and joint stiffness associated with walking are reduced;
   (5) the patient is able to move fingers more easily, such as opening a can, and perform whole body exercise, such as basketball; and
   (6) the patient shows improvement in language skills.

### Advantageous Effects of Invention

According to the present invention, for example, the pharmaceutical composition can decompose dermatan sulfate and heparan sulfate accumulated in the organ including the brain of a patient with mucopolysaccharidosis type I, particularly heparan sulfate accumulated in the central nervous system including the brain, and thus can suppress the progression of dysfunction of the organ of the patient, particularly the central nervous system including the brain.

### Brief Description of Drawings

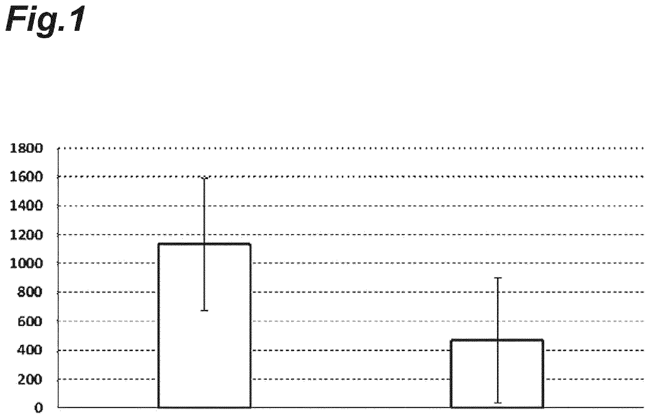

FIG. 1 is a graph showing change by administration of a test drug in a clinical trial (stage I) in the concentration of heparan sulfate contained in cerebrospinal fluids of patients with mucopolysaccharidosis type I. The bar on the left shows a value before the administration of the test drug, and the bar on the right shows a value after the administration of the test drug. The vertical axis represents the concentration (ng/mL) of heparan sulfate, and the vertical lines on the data bars represent SD bars.

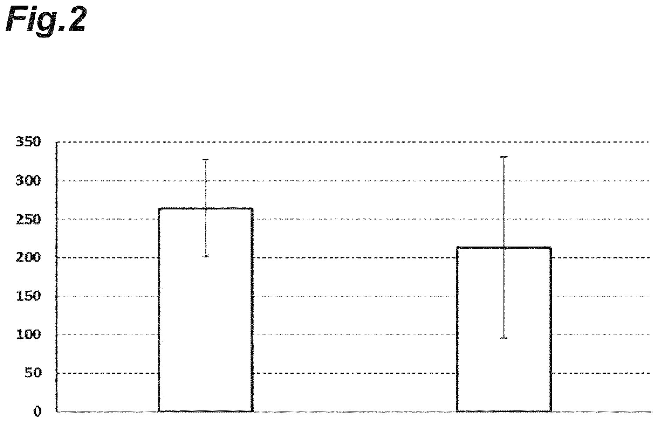

FIG. 2 is a graph showing change in the concentration of dermatan sulfate contained in cerebrospinal fluids of patients with mucopolysaccharidosis type I by administration of a test drug in a clinical trial (stage I). The bar on the left shows a value before the administration of the test drug, and the bar on the right shows a value after the administration of the test drug. The vertical axis represents the concentration (ng/mL) of dermatan sulfate, and the vertical lines on the data bars represent SD bars.

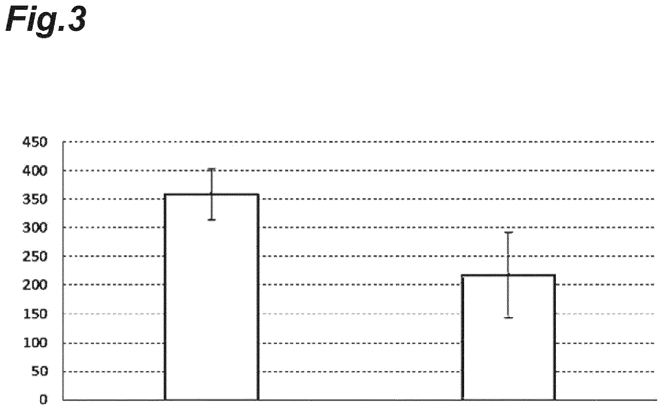

FIG. 3 is a graph showing change in the concentration of heparan sulfate contained in serums of patients with mucopolysaccharidosis type I by administration of a test drug in a clinical trial (stage I). The bar on the left shows a value before the administration of the test drug, and the bar on the right shows a value after the administration of the test drug. The vertical axis represents the concentration (ng/mL) of heparan sulfate, and the vertical lines on the data bars represent SD bars.

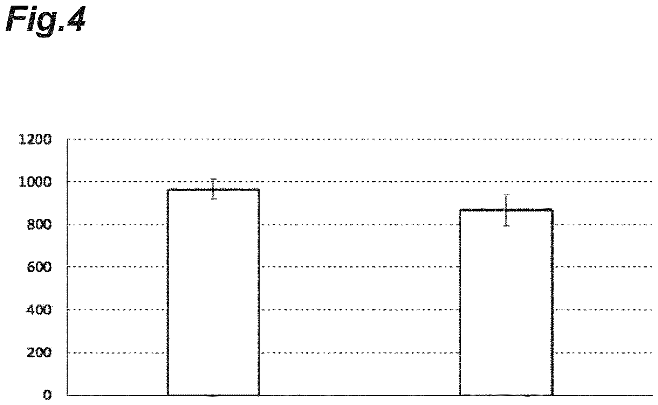

FIG. 4 is a graph showing change in the concentration of dermatan sulfate contained in serums of patients with mucopolysaccharidosis type I by administration of a test drug in a clinical trial (stage I). The bar on the left shows a value before the administration of the test drug, and the bar on the right shows a value after the administration of the test drug. The vertical axis represents the concentration (ng/mL) of dermatan sulfate, and the vertical lines on the data bars represent SD bars.

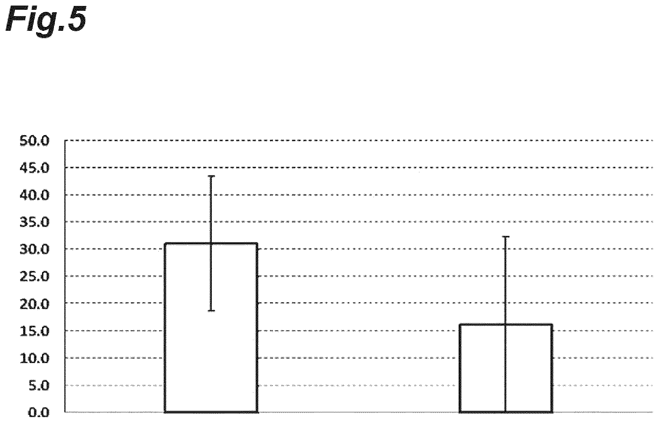

FIG. 5 is a graph showing change in the concentration of heparan sulfate contained in urines of patients with mucopolysaccharidosis type I by administration of a test drug in a clinical trial (stage I). The bar on the left shows a value before the administration of the test drug, and the bar on the right shows a value after the administration of the test drug. The vertical axis represents the amount (µg/mg creatinine) of heparan sulfate, and the vertical lines on the data bars represent SD bars.

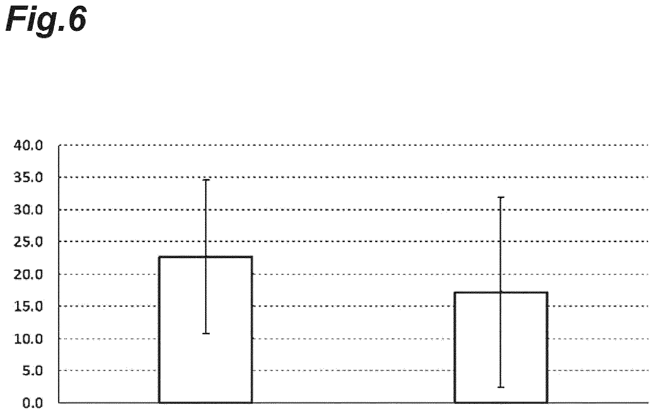

FIG. 6 is a graph showing change in the concentration of dermatan sulfate contained in urines of patients with mucopolysaccharidosis type I by administration of a test drug in a clinical trial (stage I). The graph shows a value before the administration of the test drug, and the bar on the right shows a value after the administration of the test drug. The vertical axis represents the amount (µg/mg creatinine) of dermatan sulfate, and the vertical lines on the data bars represent SD bars.

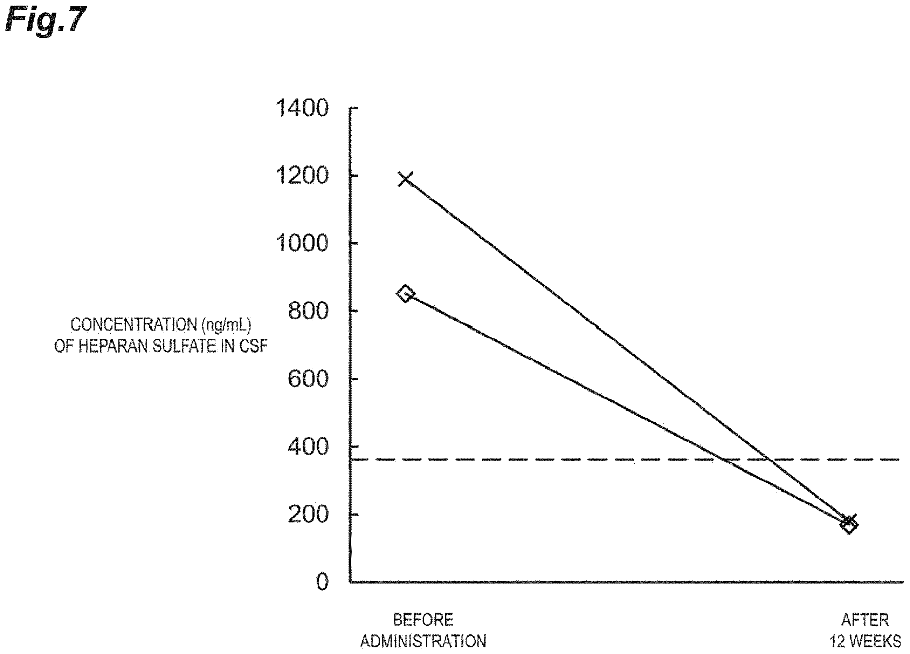

FIG. 7 is a graph showing changes in the concentration of heparan sulfate contained in cerebrospinal fluids of patients with mucopolysaccharidosis type I by administration of a test drug in a clinical trial (stage II) first trial. × and ◊ represent the concentration of heparan sulfate in each patient. The vertical axis represents the concentration (ng/mL) of heparan sulfate, and the dotted line represents an average concentration of heparan sulfate contained in cerebrospinal fluids of non-mucopolysaccharidosis type I patients.

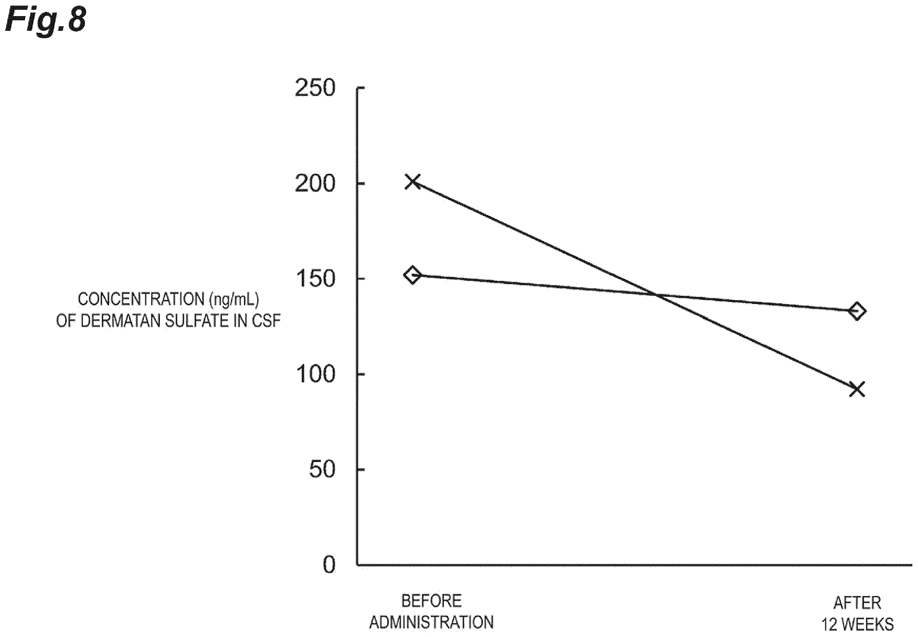

FIG. 8 is a graph showing changes in the concentration of dermatan sulfate contained in cerebrospinal fluids of patients with mucopolysaccharidosis type I by administration of a test drug in a clinical trial (stage II) first trial. × and ◊ represent the concentration of dermatan sulfate in each patient. The vertical axis represents the concentration (ng/mL) of dermatan sulfate.

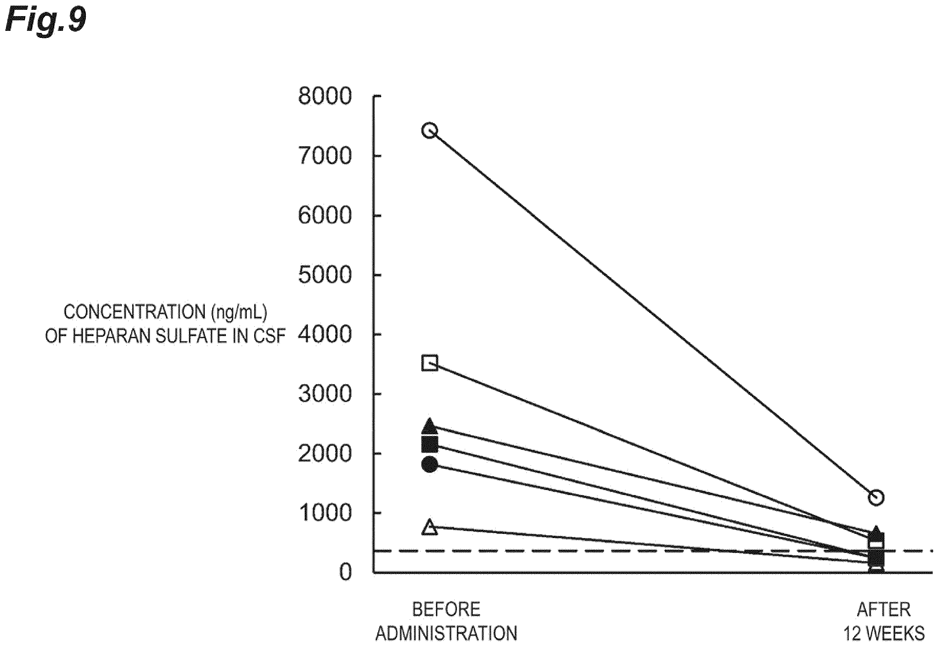

FIG. 9 is a graph showing changes in the concentration of heparan sulfate contained in cerebrospinal fluids of patients with mucopolysaccharidosis type I by administration of a test drug in a clinical trial (stage II) second trial. The white-filled symbols ○, Δ and □ represent the concentration of heparan sulfate in each patient in a 2.0 mg/kg body weight administration group, and the black-filled symbols ●, ▲, and ■ represent the concentration of heparan sulfate in each patient in a 4.0 mg/kg body weight administration group. The vertical axis represents the concentration (ng/mL) of heparan sulfate, and the dotted line represents an average concentration of heparan sulfate contained in cerebrospinal fluids of non-mucopolysaccharidosis type I patients.

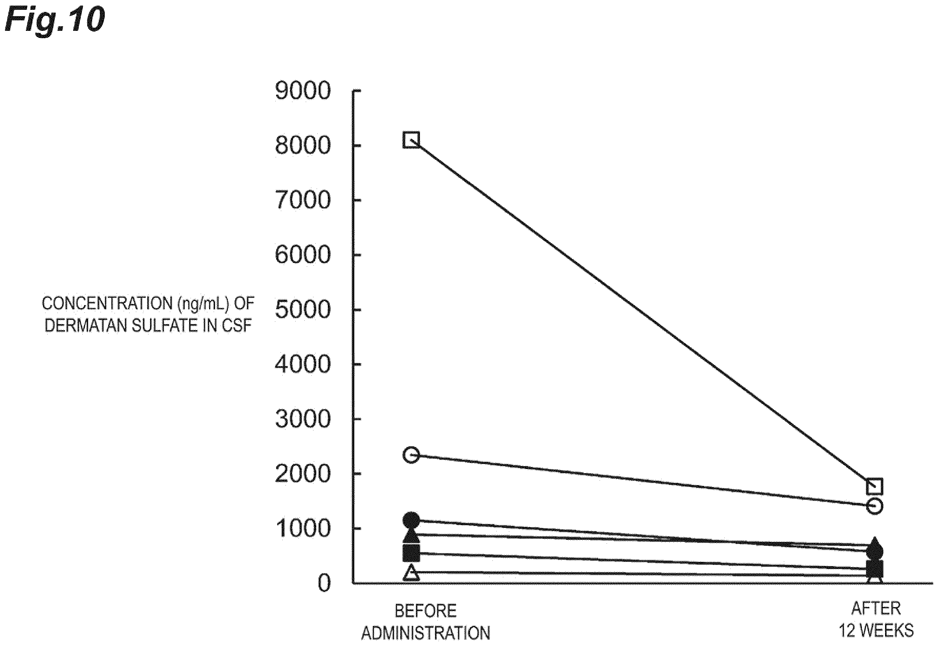

FIG. 10 is a graph showing changes in the concentration of dermatan sulfate contained in cerebrospinal fluids of patients with mucopolysaccharidosis type I by administration of a test drug in a clinical trial (stage II) second trial. The white-filled symbols o, Δ, and □ represent the concentration of dermatan sulfate in each patient in a 2.0 mg/kg body weight administration group, and the black-filled symbols ●, A, and ■ represent the concentration of dermatan sulfate in each patient in a 4.0 mg/kg body weight administration group. The vertical axis represents the concentration (ng/mL) of dermatan sulfate.

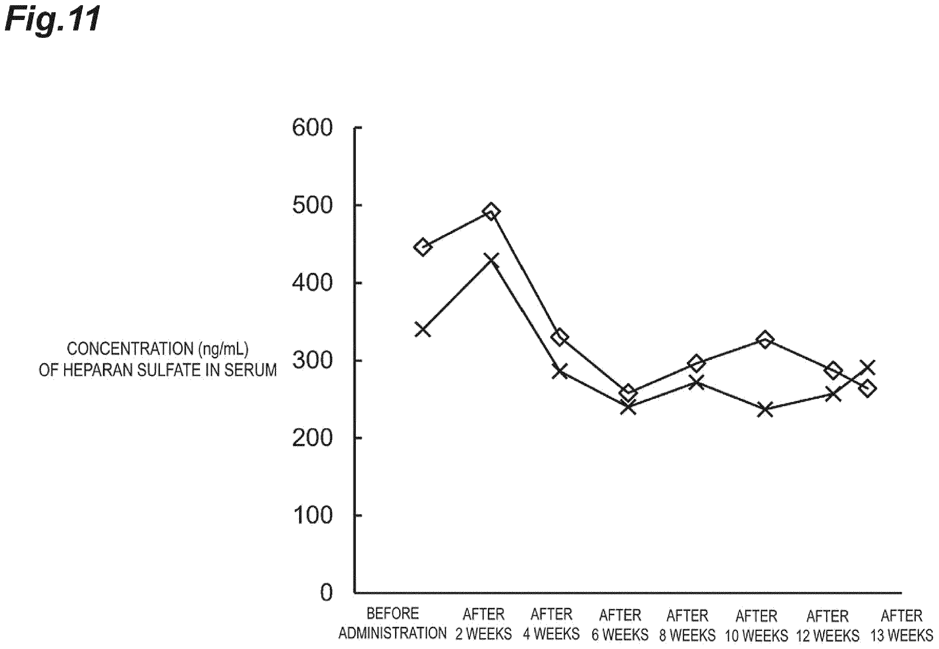

FIG. 11 is a graph showing changes in the concentration of heparan sulfate contained in serums of patients with mucopolysaccharidosis type I by administration of a test drug in a clinical trial (stage II) first trial. × and ◊ represent the concentration of heparan sulfate in each patient. The vertical axis represents the concentration (ng/mL) of heparan sulfate.

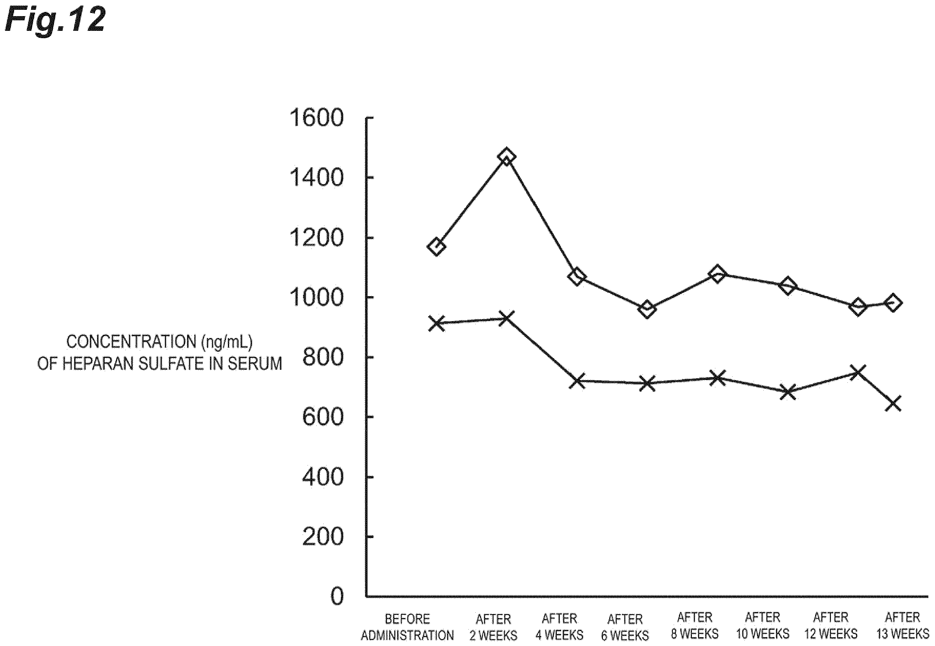

FIG. 12 is a graph showing changes in concentration of dermatan sulfate contained in serums of patients with mucopolysaccharidosis type I by administration of a test drug in a clinical trial (stage II) first trial. × and ◊ represent the concentration of dermatan sulfate in each patient. The vertical axis represents the concentration (ng/mL) of dermatan sulfate.

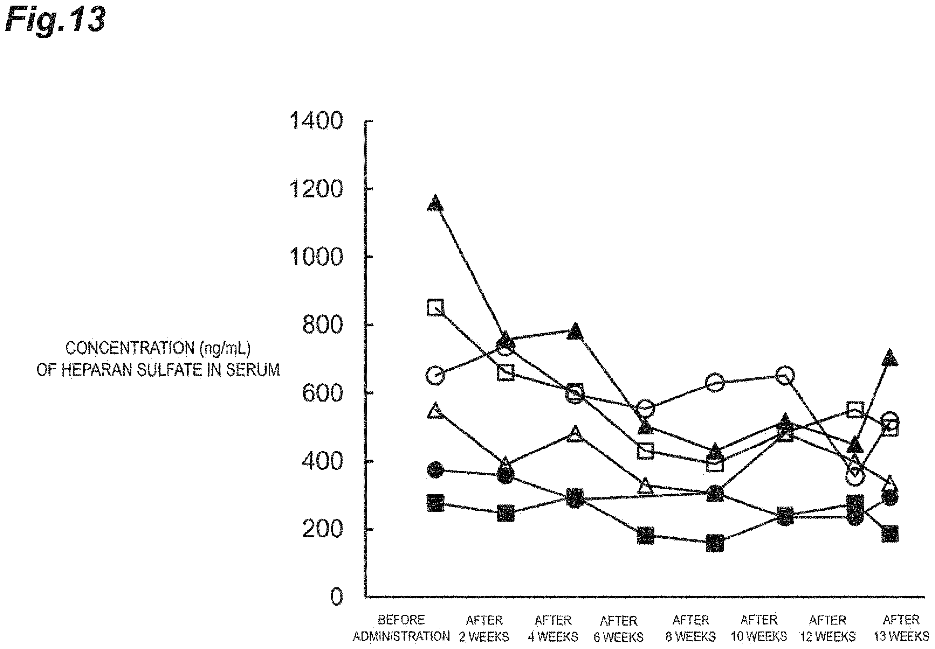

FIG. 13 is a graph showing changes in concentration of heparan sulfate contained in serums of patients with mucopolysaccharidosis type I by administration of a test drug in a clinical trial (stage II) second trial. The white-filled symbols o, Δ, and □ represent the concentration of heparan sulfate in each patient in a 2.0 mg/kg body weight administration group, and the black-filled symbols ●, ▲, and ■ represent the concentration of heparan sulfate in each patient in a 4.0 mg/kg body weight administration group. The vertical axis represents the concentration (ng/mL) of heparan sulfate.

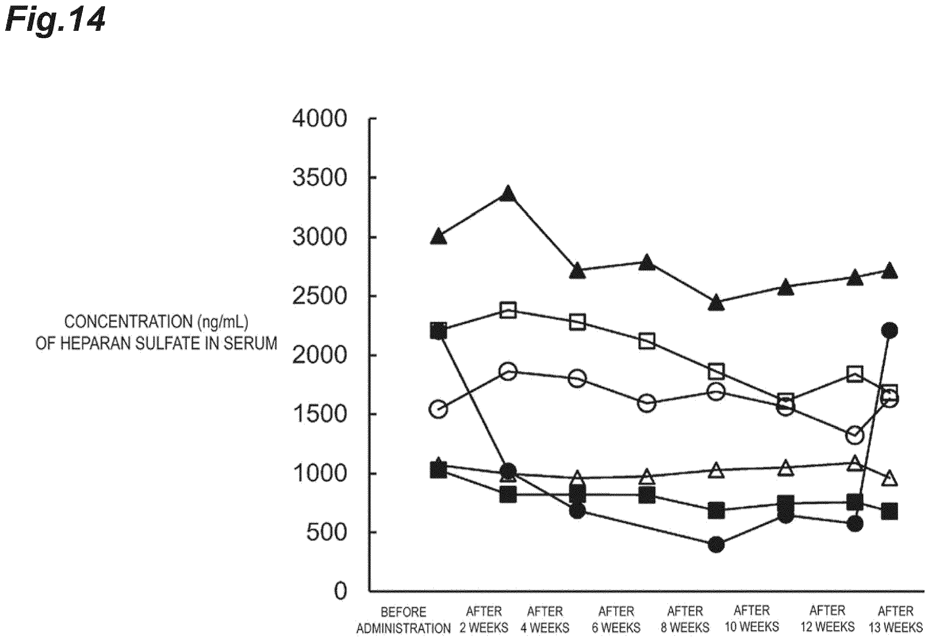

FIG. 14 is a graph showing changes in concentration of dermatan sulfate contained in serums of patients with mucopolysaccharidosis type I by administration of a test drug in a clinical trial (stage II) second trial. The white-filled symbols o, Δ, and □ represent the concentration of dermatan sulfate in each patient in a 2.0 mg/kg body weight administration group, and the black-filled symbols ●, ▲, and ■ represent the concentration of dermatan sulfate in each patient in a 4.0 mg/kg body weight administration group. The vertical axis represents the concentration (ng/mL) of dermatan sulfate.

### Description of Embodiments

A transferrin receptor (hTfR) is present on the surface of brain microvascular endothelial cells (cerebrovascular endothelial cells), which forms the human blood brain barrier. An antibody (anti-hTfR antibody) that can recognize the hTfR as an antigen can bind to the hTfR. The antibody bound to the hTfR on the surface of brain microvascular endothelial cells can cross the blood-brain barrier by hTfR-mediated transcytosis and reach the central nervous system. Thus, binding human α-L-iduronidase (hIDUA) to this antibody enables the hIDUA to reach the central nervous system.

In the present invention, the anti-hTfR antibody is preferably a Fab. Here, the Fab refers to a molecule in which one light chain containing a variable region and a constant region of the light chain (C_{L} region) and one heavy chain containing a variable region and part 1 of a constant region of the heavy chain (C_{H}1 region) are linked by a disulfide bond between cysteine residues present in each chain. In the Fab, the heavy chain may further contain part of a hinge region in addition to the variable region and the part 1 of the constant region of the heavy chain (C_{H}1 region), but in this case the hinge region lacks a cysteine residue that is otherwise present in the hinge region and links the heavy chains of the antibody to each other. In the Fab, the light chain and the heavy chain are linked by a disulfide bond formed between a cysteine residue present in the constant region (C_{L} region) of the light chain and a cysteine residue present in the constant region (C_{H}1 region) or the hinge region of the heavy chain. The heavy chain forming the Fab is referred to as a Fab heavy chain. The Fab lacks the cysteine residue, which is otherwise present in the hinge region and links the heavy chains of the antibody to each other, and thus is composed of one light chain and one heavy chain. The light chain constituting the Fab contains the variable region and the C_{L} region. The heavy chain constituting the Fab may be composed of the variable region and the C_{H}1 region or may contain part of the hinge region in addition to the variable region and the C_{H}1 region. In this case, however, the hinge region is selected to contain no cysteine residue for linking between the heavy chains and thus does not form a disulfide bond between the two heavy chains in the hinge region.

In the present invention, the anti-hTfR antibody is preferably a human antibody or a humanized antibody. The human antibody refers to an antibody that is entirely encoded by a human-derived gene. Note that an antibody encoded by a gene obtained by mutating an original human gene for the purpose, for example, of increasing the expression efficiency of the gene is also a human antibody. In addition, an antibody in which a part of one human antibody is replaced with a part of another human antibody by combining two or more genes encoding the human antibody is also a human antibody. The human antibody has three complementarity determining regions (CDRs) of an immunoglobulin light chain and three complementarity determining regions (CDRs) of an immunoglobulin heavy chain. The three CDRs of the immunoglobulin light chain are referred to as CDR1, CDR2, and CDR3 in the order from the N-terminal side. The three CDRs of the immunoglobulin heavy chain are referred to as CDR1, CDR2, and CDR3 in the order from the N-terminal side. An antibody in which the antigen specificity, affinity, and the like of a human antibody are modified by replacing a CDR of one human antibody with a CDR of another human antibody is also a human antibody.

In the present invention, the term "humanized antibody" refers to an antibody in which the amino acid sequence of part of the variable region (e.g., in particular, all, two, or one of the CDRs) is derived from a non-human mammal and another region is derived from human. Examples of the humanized antibody include an antibody prepared by replacing the three complementarity determining regions (CDRs) of an immunoglobulin light chain and the three complementarity determining regions (CDRs) of an immunoglobulin heavy chain constituting a human antibody with CDRs of another mammal. The species of another mammal from which the CDRs to be grafted at an appropriate position of the human antibody is derived is any non-human mammal without particular limitation but is preferably a mouse, a rat, a rabbit, a horse, or a non-human primate, more preferably a mouse and a rat, and for example, a mouse.

The light chains of human antibodies and humanized antibodies include a λ chain and a κ chain. The light chain constituting the antibody may be either a λ chain or a κ chain. In addition, the heavy chains of human antibodies and humanized antibodies include a γ chain, a µ chain, an α chain, a σ chain, and an ε chain, which correspond to IgG, IgM, IgA, IgD, and IgE, respectively. The heavy chain constituting the antibody may be any of a γ chain, a µ chain, an α chain, a σ chain, and an ε chain but is preferably a γ chain. Furthermore, γ chains of the heavy chains of antibodies include a γ1 chain, a γ2 chain, a γ3 chain, and a γ4 chain, which correspond to IgG1, IgG2, IgG3, and IgG4, respectively. When the heavy chain constituting the antibody is a γ chain, the γ chain may be any of a γ1 chain, a γ2 chain, a γ3 chain, and a γ4 chain but is preferably a γ1 chain or a γ4 chain. When the antibody is a humanized antibody or a human antibody and is IgG, the light chain of the antibody may be either a λ chain or a κ chain, and the heavy chain of the antibody may be any of a γ1 chain, a γ2 chain, a γ3 chain, and a γ4 chain but is preferably a γ1 chain or a γ4 chain. Examples of a preferred antibody include an antibody in which the light chain is a κ chain and the heavy chain is a γ1 chain, and an antibody in which the light chain is a λ chain and the heavy chain is a γ1 chain.

In one aspect of the present invention, the anti-hTfR antibody includes an amino acid sequence of SEQ ID NO: 4 or SEQ ID NO: 5 as CDR1, an amino acid sequence of SEQ ID NO: 6 or SEQ ID NO: 7 or an amino acid sequence Lys-Val-Ser as CDR2, and an amino acid sequence of SEQ ID NO: 8 as CDR3 in the variable region of the light chain, and includes an amino acid sequence of SEQ ID NO: 9 or 10 as CDR1, an amino acid sequence of SEQ ID NO: 11 or 12 as CDR2, and an amino acid sequence of SEQ ID NO: 13 or 14 as CDR3 in the variable region of the heavy chain. Furthermore, the anti-human transferrin receptor antibody includes an amino acid sequence of SEQ ID NO: 15, for example, in framework region 3 of the heavy chain.

In one aspect of the present invention, the anti-hTfR antibody includes a variable region of the heavy chain including an amino acid sequence of SEQ ID NO: 16 and a variable region of the light chain including an amino acid sequence of SEQ ID NO: 17.

In one suitable aspect of the present invention, the anti-hTfR antibody is a Fab in which the light chain includes an amino acid sequence of SEQ ID NO: 18 and the heavy chain is a Fab heavy chain including an amino acid sequence of SEQ ID NO: 19.

In one aspect of the present invention, the anti-hTfR antibody may be an anti-hTfR antibody in which a mutation, such as substitution, deletion, or addition, is introduced to the amino acid sequence of the variable region or another part including the variable region as long as the amino acid sequence of each CDR is conserved and as long as the affinity for the hTfR is retained in the amino acid sequence. When an amino acid or amino acids in the amino acid sequence of the variable region of the anti-hTfR antibody is/are substituted with another amino acid or other amino acids, the number of amino acid(s) to be substituted is preferably from 1 to 10, more preferably from 1 to 5, even more preferably from 1 to 3, and still more preferably 1 or 2. In deletion of an amino acid or amino acids in the amino acid sequence of the variable region of the anti-hTfR antibody, the number of amino acid(s) to be deleted is preferably from 1 to 10, more preferably from 1 to 5, even more preferably from 1 to 3, and still more preferably 1 or 2. In addition, a mutation combining substitution and deletion of these amino acids can also be performed. In adding an amino acid or amino acids to the variable region of the anti-hTfR antibody, preferably from 1 to 10, more preferably from 1 to 5, even more preferably from 1 to 3, and even more preferably 1 or 2 amino acids are added in the amino acid sequence of the variable region, or on the N-terminal side or the C-terminal side of the anti-hTfR antibody. A mutation combining addition, substitution, and deletion of these amino acids can also be performed. The amino acid sequence of the variable region of the mutated anti-hTfR antibody preferably has 80% or more identity with the amino acid sequence of the variable region of the original anti-hTfR antibody, and more preferably shows 85% or more identity, even more preferably 90% or more identity, still more preferably 95% or more identity, and yet more preferably 99% or more identity with the amino acid sequence of the variable region of the original anti-hTfR antibody. The same applies to a case of performing a mutation to the amino acid sequence of another part including the variable region. Furthermore, the Kd value for the hTfR of the mutated anti-hTfR antibody is desirably a value 50 times or less compared with that of the original anti-hTfR antibody, and for example, preferably a value 10 times or less.

In one aspect of the present invention, the anti-hTfR antibody has affinity for both the extracellular region of the hTfR and the extracellular region of the monkey TfR. In that case, the dissociation constant of the anti-hTfR antibody with the extracellular region of the hTfR is preferably 1 × 10⁻¹⁰ M or less, and the dissociation constant with the extracellular region of the monkey TfR is preferably 5 × 10⁻⁹ M or less.

In one aspect of the present invention, the term "human α-L-iduronidase" or "hIDUA" refers particularly to hIDUA with the same amino acid sequence as that of wild-type hIDUA. The wild-type hIDUA has an amino acid sequence composed of 628 amino acids represented by SEQ ID NO: 20. A variant of the hIDUA, the variant with an amino acid sequence composed of 626 amino acids represented by SEQ ID NO: 21, is also hIDUA. However, the hIDUA is not limited to these and includes those obtained by introducing a mutation, such as substitution, deletion, and addition, to the amino acid sequence of the wild-type hIDUA as long as it has IDUA activity. In substitution of one or more amino acids in the amino acid sequence of the hIDUA with another amino acid or other amino acids, the number of amino acid(s) to be substituted is preferably from 1 to 10, more preferably from 1 to 5, even more preferably from 1 to 3, and still more preferably 1 or 2. In deletion of one or more amino acids in the amino acid sequence of the hIDUA, the number of amino acid(s) to be deleted is preferably from 1 to 10, more preferably from 1 to 5, even more preferably from 1 to 3, and still more preferably 1 or 2. In addition, a mutation combining substitution and deletion of these amino acids can also be performed. In adding one or more amino acids to the hIDUA, preferably from 1 to 10, more preferably from 1 to 5, even more preferably from 1 to 3, and even more preferably 1 or 2 amino acids are added in the amino acid sequence, or on the N-terminal side or the C-terminal side of the hIDUA. A mutation combining addition, substitution, and deletion of these amino acids can also be performed. The amino acid sequence of the mutated hIDUA preferably has 80% or more identity with the amino acid sequence of the original hIDUA, and more preferably shows 85% or more identity, even more preferably 90% or more identity, still more preferably 95% or more identity, and yet more preferably 99% or more identity with the amino acid sequence of the original hIDUA.

In the present invention, when hIDUA is referred to as having IDUA activity, this means that when the hIDUA is fused with an antibody to form a fusion protein, the fusion protein has an activity that is 3% or more of the activity originally possessed by the native hIDUA. However, the activity is preferably 10% or more, more preferably 20% or more, even more preferably 50% or more, and still more preferably 80% or more of the activity originally possessed by the native hIDUA. The same applies to a case where the hIDUA fused with an antibody is mutated. The antibody is, for example, an anti-hTfR antibody.

In the present invention, the identity of the amino acid sequence of an original protein (including an antibody) with the amino acid sequence of a mutated protein can be easily calculated using a known identity calculation algorithm. Examples of such an algorithm include BLAST (Altschul SF. J Mol. Biol. 215. 403-10, (1990)), the similarity search method of Pearson and Lipman (Proc. Natl. Acad. Sci. USA. 85. 2444 (1988)), and the local identity algorithm of Smith and Waterman (Adv. Appl. Math. 2. 482-9 (1981)).

Furthermore, substitution of an amino acid in the amino acid sequence of an original protein (including an antibody) with another amino acid can occur, for example, in an amino acid family including amino acids relevant each other to their side chains and chemical properties. Such a substitution in a family of amino acids is expected not to result in a significant change in the function of the original protein (i.e., to be a conservative amino acid substitution). Examples of such an amino acid family include the following:
(1) aspartic acid and glutamic acid, which are acidic amino acids;
(2) histidine, lysine, and arginine, which are basic amino acids;
(3) phenylalanine, tyrosine, and tryptophan, which are aromatic amino acids;
(4) serine and threonine, which are amino acids with a hydroxyl group (hydroxyamino acids);
(5) methionine, alanine, valine, leucine, and isoleucine, which are hydrophobic amino acids;
(6) cysteine, serine, threonine, asparagine, and glutamine, which are neutral hydrophilic amino acids;
(7) glycine and proline, which are amino acids affecting the orientation of peptide chains;
(8) asparagine and glutamine, which are amide amino acids (polar amino acids);
(9) alanine, leucine, isoleucine, and valine, which are aliphatic amino acids;
(10) alanine, glycine, serine, and threonine, which are amino acids with a small side-chain;
(11) alanine and glycine, which are amino acids with a particularly small side chain; and
(12) valine, leucine, and isoleucine, which are amino acids with a branched chain.

In one aspect of the present invention, the fusion protein of an anti-hTfR antibody and hIDUA refers to a substance in which the anti-hTfR antibody and the hIDUA are linked via a peptide linker or directly. For example, the fusion protein is a fusion protein of an anti-hTfR antibody and hIDUA, or a substance in which the N-terminus or C-terminus of the hIDUA is linked respectively to the C-terminus or N-terminus of the heavy chain or light chain of the antibody by a peptide bond via a linker or directly.

A fusion protein of the type in which hIDUA is linked to the C-terminus of the light chain of the hTfR antibody is a fusion protein in which the antibody includes an amino acid sequence including all or part of the variable region of the light chain and an amino acid sequence including all or part of the variable region of the heavy chain (e.g., a Fab heavy chain), and the hIDUA is linked to the C-terminus of the light chain of this antibody. Here, the light chain of the antibody and the hIDUA may be linked directly or may be linked via a linker.

A fusion protein of the type in which hIDUA is linked to the C-terminus of the heavy chain of the hTfR antibody is a fusion protein in which the antibody includes an amino acid sequence including all or part of the variable region of the light chain and an amino acid sequence including all or part of the variable region of the heavy chain (e.g., a Fab heavy chain), and the hIDUA is linked to the C-terminus of the heavy chain of this antibody. Here, the heavy chain of the antibody and the hIDUA may be linked directly or may be linked via a linker.

A fusion protein of the type in which hIDUA is linked to the N-terminus of the light chain of the hTfR antibody is a fusion protein in which the antibody includes an amino acid sequence including all or part of the variable region of the light chain and an amino acid sequence including all or part of the variable region of the heavy chain (e.g., a Fab heavy chain), and the hIDUA is linked to the N-terminus of the light chain of this antibody. Here, the light chain of the antibody and the hIDUA may be linked directly or may be linked via a linker.

A fusion protein of the type in which hIDUA is linked to the N-terminus of the heavy chain of the hTfR antibody is a fusion protein in which the antibody includes an amino acid sequence including all or part of the variable region of the light chain and an amino acid sequence including all or part of the variable region of the heavy chain (e.g., a Fab heavy chain), and the hIDUA is linked to the N-terminus of the heavy chain of this antibody. Here, the heavy chain of the antibody and the hIDUA may be linked directly or may be linked via a linker.

In this case, when a linker is disposed between the hTfR antibody and the hIDUA, its sequence is composed preferably of 1 to 50 amino acids, more preferably of 1 to 20 amino acids, even more preferably of 10 to 17 amino acids, still more preferably of 13 to 17 amino acids, and for example, 15 amino acids. The amino acid sequence of such a linker is not limited as long as the antibody linked by the linker retains the affinity for the hTfR, and the hIDUA linked by the linker can exhibit the biological activity of the protein under physiological conditions. However, the linker is preferably composed of glycine and serine, for example, composed of one amino acid of either glycine or serine, or has an amino acid sequence Gly-Ser, an amino acid sequence Gly-Gly-Ser, an amino acid sequence represented by SEQ ID NO: 1, an amino acid sequence represented by SEQ ID NO: 2, an amino acid sequence represented by SEQ ID NO: 3, or a sequence composed of 1 to 150 amino acids composed successively of 1 to 10 or of 2 to 5 of these amino acid sequences, or a sequence composed of 2 to 17, 2 to 10, 10 to 40, 20 to 34, 23 to 31, or 25 to 29 amino acids. For example, an entity composed of the amino acid sequence Gly-Ser or having the amino acid sequence represented by SEQ ID NO: 3 can be suitably used as the linker.

Suitable examples of the fusion protein of an anti-hTfR antibody and hIDUA in the present invention include a fusion protein in which
the light chain of the anti-hTfR antibody includes the amino acid sequence of SEQ ID NO: 18,
the heavy chain of the anti-hTfR antibody includes the amino acid sequence of SEQ ID NO: 19, and
the heavy chain is linked at its C-terminus to the human α-L-iduronidase with the amino acid sequence of SEQ ID NO: 20 or SEQ ID NO: 21 via a linker of the amino acid sequence of SEQ ID NO: 3.

Further suitable examples of the fusion protein of an anti-hTfR antibody and hIDUA in the present invention include a fusion protein in which
the light chain of the anti-hTfR antibody includes the amino acid sequence of SEQ ID NO: 18,
the heavy chain of the anti-hTfR antibody includes the amino acid sequence of SEQ ID NO: 19, and
the heavy chain is linked at its C-terminus to the human α-L-iduronidase with the amino acid sequence of SEQ ID NO: 20 via the linker of the amino acid sequence of SEQ ID NO: 3, thereby to form an amino acid sequence of SEQ ID NO: 24.

Further suitable examples of the fusion protein of an anti-hTfR antibody and hIDUA in the present invention include a fusion protein in which
the light chain of the anti-hTfR antibody consists of the amino acid sequence of SEQ ID NO: 18,
the heavy chain of the anti-hTfR antibody consists of the amino acid sequence of SEQ ID NO: 19, and
the heavy chain is linked at its C-terminal side to the human α-L-iduronidase with the amino acid sequence of SEQ ID NO: 20 or SEQ ID NO: 21 via the linker of the amino acid sequence of SEQ ID NO: 3.

A pharmaceutical composition in the present invention contains, as an active ingredient, a fusion protein of an anti-hTfR antibody and hIDUA. The pharmaceutical composition may be a lyophilized preparation or an aqueous liquid preparation.

The pharmaceutical composition in one embodiment of the present invention contains at least one selected from the group consisting of a neutral salt, a disaccharide, a nonionic surfactant, and a buffer. The pharmaceutical may further contain a polysorbate and/or a poloxamer as a nonionic surfactant.

The nonionic surfactant contained in the pharmaceutical composition is any pharmaceutically acceptable nonionic surfactant without particular limitation, but such nonionic surfactants are suitably a polysorbate and a poloxamer. Here, the polysorbate can be exemplified by polysorbate 20 and polysorbate 80. In addition, the poloxamer can be exemplified by poly(oxyethylene) (54) poly(oxypropylene) (39) glycol, poly(oxyethylene) (196) poly(oxypropylene) (67) glycol, poly(oxyethylene) (42) poly(oxypropylene) (67) glycol, poly(oxyethylene) (3) poly(oxypropylene) (17) glycol, poly(oxyethylene) (20) poly(oxypropylene) (20) glycol, and poly(oxyethylene) (120) poly(oxypropylene) (40) glycol, and poly(oxyethylene) (160) poly(oxypropylene) (30) glycol is particularly suitable. Poly(oxyethylene) (160) poly(oxypropylene) (30) glycol is synonymous with poloxamer 188.

The pharmaceutical composition may contain two nonionic surfactants. A preferred combination of nonionic surfactants for the pharmaceutical composition containing two nonionic surfactants is a combination in which one is a polysorbate and the other is a poloxamer. For example, a combination of polysorbate 20 and poloxamer 188 or a combination of polysorbate 80 and poloxamer 188 is preferred, and a combination of polysorbate 80 and poloxamer 188 is particularly preferred. A combination of these can be further combined with another type of polysorbate, poloxamer, or the like.

In a case where the pharmaceutical composition is an aqueous liquid preparation in which the aqueous liquid preparation contains a polysorbate and a poloxamer as two nonionic surfactants, the concentration of the polysorbate is preferably from 0.005 to 1.5 mg/mL, more preferably from 0.025 to 1.0 mg/mL, even more preferably from 0.05 to 1.0 mg/mL, still more preferably from 0.05 to 0.15 mg/mL, and for example, 0.075 mg/mL. In addition, the concentration of the poloxamer in this case is preferably from 0.1 to 0.6 mg/mL, more preferably from 0.2 to 0.5 mg/mL, even more preferably from 0.25 to 0.45 mg/mL, and for example, 0.325 mg/mL.

In a case where the pharmaceutical composition is an aqueous liquid preparation in which the aqueous liquid preparation contains polysorbate 80 and poloxamer 188 as two nonionic surfactants, the concentration of polysorbate 80 is preferably from 0.005 to 1.5 mg/mL, more preferably from 0.025 to 1.0 mg/mL, even more preferably from 0.05 to 1.0 mg/mL, and for example, 0.075 mg/mL. In addition, the concentration of poloxamer 188 in this case is preferably from 0.1 to 0.6 mg/mL, more preferably from 0.2 to 0.5 mg/mL, even more preferably from 0.25 to 0.45 mg/mL, and for example, 0.325 mg/mL. For example, the concentration of polysorbate 80 is from 0.05 to 1.0 mg/mL, and the concentration of poloxamer 188 is from 0.25 to 0.45 mg/mL. Furthermore, for example, the concentration of polysorbate 80 is 0.075 mg/mL, and the concentration of poloxamer 188 is 0.325 mg/mL.

The neutral salt contained in the pharmaceutical composition is any pharmaceutically acceptable neutral salt without particular limitation, but such a neutral salt is suitably sodium chloride or magnesium chloride and particularly suitably sodium chloride.

The disaccharide contained in the pharmaceutical composition is any pharmaceutically acceptable disaccharide without particular limitation, but such a disaccharide is suitably trehalose, sucrose, maltose, lactose, or a combination of these, and particularly suitably sucrose.

In a case where the pharmaceutical composition is an aqueous liquid preparation, the concentration of the disaccharide in the aqueous liquid preparation is preferably from 50 to 100 mg/mL, more preferably from 55 to 95 mg/mL, even more preferably from 60 to 90 mg/mL, and for example, 75 mg/mL.

The buffer contained in the pharmaceutical composition is any pharmaceutically acceptable buffer without particular limitation but preferably a citrate buffer, a phosphate buffer, a glycine buffer, a histidine buffer, a carbonate buffer, an acetate buffer, or a combination of these. In a case where the pharmaceutical composition is an aqueous liquid preparation, the concentration of the buffer contained in the aqueous liquid preparation is preferably from 3 to 30 mM, more preferably from 10 to 30 mM, even more preferably from 15 to 25 mM, and for example, 20 mM. In a case where the aqueous liquid preparation in which a citrate buffer is used as the buffer, the concentration of the citrate buffer contained in the aqueous liquid preparation is preferably from 3 to 30 mM, more preferably from 10 to 30 mM, even more preferably from 15 to 25 mM, and for example, 20 mM. In addition, the pH of the aqueous liquid preparation adjusted with a buffer is preferably from 4.5 to 7.0, more preferably from 4.5 to 6.5, even more preferably from 5.0 to 6.0, still more preferably from 5.2 to 5.8, and for example, 5.5. Furthermore, the pH of the aqueous liquid preparation adjusted with a citrate buffer is preferably from 4.5 to 7.0, more preferably from 4.5 to 6.5, even more preferably from 5.0 to 6.0, still more preferably from 5.2 to 5.8, and for example, 5.5.

Examples of a suitable pharmaceutical composition as an aqueous liquid preparation include (1) to (3) below:
(1) an aqueous liquid preparation with a concentration of the fusion protein of 1 to 10 mg/mL, a concentration of the neutral salt of 0.3 to 1.2 mg/mL, a concentration of the disaccharide of 50 to 100 mg/mL, a concentration of the buffer of 10 to 30 mM, a concentration of the polysorbate of 0.005 to 1.5 mg/mL, and a concentration of the poloxamer of 0.1 to 0.6 mg/mL;
(2) an aqueous liquid preparation with a concentration of the fusion protein of 2 to 8 mg/mL, a concentration of the neutral salt of 0.5 to 1.0 mg/mL, a concentration of the disaccharide of 55 to 95 mg/mL, a concentration of the buffer of 15 to 25 mM, a concentration of the polysorbate of 0.05 to 1.0 mg/mL, and a concentration of the poloxamer of 0.25 to 0.45 mg/mL; and
(3) an aqueous liquid preparation with a concentration of the fusion protein of 4 to 6 mg/mL, a concentration of the neutral salt of 0.7 to 0.9 mg/mL, a concentration of the disaccharide of 60 to 90 mg/mL, a concentration of the buffer of 15 to 25 mM, a concentration of the polysorbate of 0.05 to 0.15 mg/mL, and a concentration of the poloxamer of 0.25 to 0.45 mg/mL.

The pH of the aqueous liquid preparations of (1) to (3) above is adjusted to a pH, for example, from 4.5 to 6.5, from 5.0 to 6.0, or from 5.2 to 5.8.

Examples of a suitable pharmaceutical composition being a lyophilized preparation include (1) to (3) below:
(1) a lyophilized preparation giving a concentration of the fusion protein of 1 to 10 mg/mL, a concentration of the neutral salt of 0.3 to 1.2 mg/mL, a concentration of the disaccharide of 50 to 100 mg/mL, a concentration of the buffer of 10 to 30 mM, a concentration of the polysorbate of 0.005 to 1.5 mg/mL, and a concentration of the poloxamer of 0.1 to 0.6 mg/mL when dissolved in pure water;
(2) a lyophilized preparation giving a concentration of the fusion protein of 2 to 8 mg/mL, a concentration of the neutral salt of 0.5 to 1.0 mg/mL, a concentration of the disaccharide of 55 to 95 mg/mL, a concentration of the buffer of 15 to 25 mM, a concentration of the polysorbate of 0.05 to 1.0 mg/mL, and a concentration of the poloxamer of 0.25 to 0.45 mg/mL when dissolved in pure water; and
(3) a lyophilized preparation giving a concentration of the fusion protein of the neutral salt of 4 to 6 mg/mL, a concentration of 0.7 to 0.9 mg/mL, a concentration of the disaccharide of 60 to 90 mg/mL, a concentration of the buffer of 15 to 25 mM, a concentration of the polysorbate of 0.05 to 0.15 mg/mL, and a concentration of the poloxamer of 0.25 to 0.45 mg/mL when dissolved in pure water.

The pH when the lyophilized preparation of (1) to (3) is dissolved in pure water is, for example, from 4.5 to 6.5, from 5.0 to 6.0, or from 5.2 to 5.8.

The pharmaceutical composition being an aqueous liquid preparation may be filled in a vial or can be supplied as a prefilled preparation, in which the aqueous liquid preparation is prefilled in a syringe in advance. The material of the container, such as a syringe and a vial, to be filled with the aqueous pharmaceutical composition is not particularly limited but is preferably a material made of borosilicate glass. In addition to this, a material made of a hydrophobic resin, such as a cycloolefin copolymer, which is a copolymer of a cyclic olefin and an olefin, a ring-opened polymer of a cycloolefin, or a hydrogenated ring-opened polymer of a cycloolefin, is also suitable.

The pharmaceutical composition being a lyophilized preparation can also be supplied as a kit together with a dedicated solution for dissolving this. The lyophilized preparation is dissolved, for example, in the dedicated solution or pure water before use. The material of the container, such as a syringe and a vial, for sealing the lyophilized formulation or to be filled with the lyophilized formulation is not particularly limited but is preferably a material made of borosilicate glass. In addition, a material made of a hydrophobic resin, such as a cycloolefin copolymer, which is a copolymer of a cyclic olefin and an olefin, a ring-opened polymer of a cycloolefin, or a hydrogenated ring-opened polymer of a cycloolefin, is also suitable.

The pharmaceutical composition, whether in the form of an aqueous liquid preparation or a lyophilized preparation, is usually added to a dialysis bag containing physiological saline or the like, diluted, and injected into a patient.

In one embodiment of the present invention, the pharmaceutical composition is for use as a therapeutic agent for mucopolysaccharidosis type I. Mucopolysaccharidosis type I is classified into severe Hurler syndrome (MPS IH), intermediate Hurler-Scheie syndrome (MPS IH-S), and mild Scheie syndrome (MPS IS). The pharmaceutical composition can be used for any type of mucopolysaccharidosis type I.

Mucopolysaccharidosis type I is caused by a partial or complete genetic deficiency of hIDUA, which has an activity of hydrolyzing non-sulfated α-L-iduronosidic linkages present in molecules of glycosaminoglycans (GAGs), such as heparan sulfate and dermatan sulfate. The patients present with various symptoms, such as skeletal deformities and severe mental retardation, due to abnormal accumulation of heparan sulfate and dermatan sulfate in the tissues throughout the body, including the brain, caused by deficiency of hIDUA in mucopolysaccharidosis type I.

The fusion protein of an anti-hTfR antibody and hIDUA, the active ingredient of the pharmaceutical composition, can cross the BBB and exhibit IDUA activity in brain tissue, and can decompose heparan sulfate and dermatan sulfate. Thus, the pharmaceutical composition is effective as a therapeutic agent for use in enzyme replacement therapy for a patient with mucopolysaccharidosis type I particularly with a disorder in the central nervous system.

In one embodiment of the present invention, the pharmaceutical composition is administered to a patient with mucopolysaccharidosis type I by a parenteral route, for example, by intravenous infusion, for example, infused intravenously by drip.

The pharmaceutical composition is administered to a patient with mucopolysaccharidosis type I by intravenous infusion at a dose of 0.1 to 10 mg/kg body weight, for example, at a dose of 1 to 10 mg/kg body weight, 1 to 6 mg/kg body weight, of 2 to 6 mg/kg body weight, 2 mg/kg body weight, 4 mg/kg body weight, 6 mg/kg body weight, or 8 mg/kg body weight. When the pharmaceutical composition is infused intravenously by drip, the dose rate is adjusted to infuse the fusion protein in an amount of 0.33 mg to 200 mg per hour. The time required for administration is usually from 30 minutes to 4 hours, and for example, for 3 hours.

Mucopolysaccharidosis type I is a genetic disease, and the administration is a symptomatic therapy, and thus the pharmaceutical composition needs to be administered continually. The pharmaceutical composition is administered at intervals preferably of 3 to 21 days, more preferably of 5 to 14 days, for example, at intervals of 7 days, or 14 days. The continual administration period of the pharmaceutical composition is not particularly limited but is preferably for at least 1 month and more preferably for at least 3 months. The pharmaceutical composition is assumed to be administered over a lifetime.

The dose of the pharmaceutical composition can be kept small in a first administration and then gradually increased. The active ingredient, the fusion protein of an anti-hTfR antibody and hIDUA, is a foreign substance to patients and thus may cause an adverse reaction, such as an immune reaction. Employing the method of gradually increasing can reduce the risk of causing a rapid adverse reaction in patients.

Examples of the administration schedule when the method of gradually increasing the dose is employed include (1) to (8) below:
(1) administered at a dose of 0.1 to 2 mg/kg body weight in a first administration, and then the dose is increased compared with the first dose;
(2) administered at a dose of 0.1 to 2 mg/kg body weight in a first administration, and then the dose is increased to 2 mg/kg body weight, 4 mg/kg body weight, or 6 mg/kg body weight;
(3) administered at a dose of 0.1 to 0.5 mg/kg body weight in a first administration, at a dose of 1 to 2 mg/kg body weight in second and third administrations, and at a maintenance dose of 4 mg/kg body weight in fourth and subsequent administrations;
(4) administered at a dose of 0.1 to 0.2 mg/kg body weight in a first administration, at a dose of 2 mg/kg body weight in a second administration, and at a dose of 4 mg/kg body weight (maintenance dose) in third and subsequent administrations;
(5) administered at a dose of 0.1 mg/kg body weight in a first administration and at a dose of 2 mg/kg body weight (maintenance dose) in second and subsequent administrations;
(6) administered at a dose of 0.1 mg/kg body weight in a first administration, at a dose of 1 mg/kg body weight in a second administration, at a dose of 2 mg/kg body weight in a third administration, and at a dose of 4 mg/kg body weight (maintenance dose) in fourth and subsequent administrations;
(7) administered at a dose of 0.1 mg/kg body weight in a first administration, at a dose of 2 mg/kg body weight in a second administration, and at a dose of 4 mg/kg body weight (maintenance dose) in fourth and subsequent administrations;
(8) administered at a dose of 1.0 mg/kg body weight in a first administration, at a dose of 2 mg/kg body weight in a second administration, and at a dose of 4 mg/kg body weight (maintenance dose) in fourth and subsequent administrations.

According to a suitable dosage and administration of the pharmaceutical composition, infusion is conducted intravenously into a patient by drip at a dose of 4 mg/kg body weight for at least 1 month at intervals of 7 days over at least 1 or 2 hours, for example, 3 hours.

The pharmaceutical composition does not cause serious adverse events in a patient when administered to the patient according to the dosage and administration described above. However, to suppress immune reaction, the pharmaceutical composition can be used in combination with an immunosuppressive agent. The immunosuppressive agent that can be used in combination is not particularly limited; for example, an alkylating agent, such as cyclophosphamide; an antimetabolite, such as azathioprine, mycophenolate mofetil, methotrexate, or mizoribine; or an intracellular signaling inhibitor, such as cyclosporine or tacrolimus; can be used.

When the pharmaceutical composition is used as a therapeutic agent for use in enzyme replacement therapy for a patient with mucopolysaccharidosis type I, the effect of administration of the pharmaceutical composition can be evaluated, for example, by collecting cerebrospinal fluid (CSF), serum, and/or urine of the patient before and after the administration, and measuring the concentration of heparan sulfate and/or dermatan sulfate in these. The accumulation of heparan sulfate in the central nervous system is believed to be responsible for the dysfunction of the central nervous system. Thus, a decrease in the concentration of heparan sulfate in the cerebrospinal fluid is expected to improve the function of the central nervous system of the patient. The concentration of heparan sulfate and/or dermatan sulfate can be measured by a method described in Examples.

When the pharmaceutical composition is administered to a patient with mucopolysaccharidosis type I, the concentration of heparan sulfate contained in cerebrospinal fluid collected from the patient after the administration decreases compared with the concentration of heparan sulfate contained in cerebrospinal fluid collected from the patient before the first administration of the pharmaceutical composition. The concentration of heparan sulfate after the administration is preferably 2/3 or less, 1/2 or less, or 1/3 or less compared with the concentration of heparan sulfate before the administration.

For example, the pharmaceutical composition can reduce the concentration of heparan sulfate and/or dermatan sulfate in the cerebrospinal fluid (CSF), serum, and/or urine of a patient with mucopolysaccharidosis type I when the pharmaceutical composition is administered to a patient with mucopolysaccharidosis type I at a dose of 2 mg/kg body weight or 4 mg/kg body weight, or administering for 3 months (12 weeks) at intervals of 7 days by gradually increasing a dose from a dose of 1.0 mg/kg body weight in a first administration, to a dose of 2 mg/kg body weight in a second administration, and to a dose of 4 mg/kg body weight (maintenance dose) in fourth and subsequent administrations. When the pharmaceutical composition is administered to a patient according to such dosage and administration, the concentration of heparan sulfate contained in cerebrospinal fluid collected from the patient after the administration is preferably 2/3 or less, more preferably 1/2 or less, even more preferably 1/3 or less compared with the concentration of heparan sulfate contained in cerebrospinal fluid collected from the patient before the first administration of the pharmaceutical composition. Alternatively, the concentration of heparan sulfate contained in cerebrospinal fluid collected from the patient after the administration is preferably 2000 ng/mL or less, more preferably 1800 ng/mL or less, even more preferably 1600 ng/mL or less, still more preferably 1500 ng/mL or less, and yet more preferably 1400 ng/mL or less.

The pharmaceutical composition can be expected to ameliorate dysfunction of a patient with mucopolysaccharidosis type I when the pharmaceutical composition is administered to a patient with mucopolysaccharidosis type I. Examples of expected amelioration of dysfunction include, but are not limited to, ability to have a longer conversation; ability to write more letters and/or characters; an increase in vitality; reduction in joint pain in the lower back, knees, and the like; reduction of pain and muscle and joint stiffness associated with walking; improved mobility of fingers, such as opening a can, and ability to perform whole body exercise, such as basketball; and improvement in language skills.

### Examples

Hereinafter, the present invention will be described in more detail with reference to examples, but the present invention is not intended to be limited to the examples.

### Example 1: Construction of vector for expression of humanized anti-hTfR antibody-hlDUA fusion protein

A vector for expression of a humanized anti-hTfR antibody-hIDUA fusion protein was constructed using a gene encoding, as the antibody part, the light chain with the amino acid sequence represented by SEQ ID NO: 18 and the Fab region of the heavy chain with the amino acid sequence represented by SEQ ID NO: 19.

### Construction of pE-neo vector and pE-hygr vector

A pEF/myc/nuc vector (Invitrogen) was digested with KpnI and NcoI, the region containing the EF-1 promoter and its first intron was excised, and this was blunt-ended with T4 DNA polymerase. Separately, pCI-neo (Invitrogen) was digested with BglII and EcoRI to cut off the region containing the enhancer/promoter and intron of CMV and then blunt-ended with T4 DNA polymerase. Into this, the region (blunt-ended region) containing the EF-1α promoter and its first intron was inserted, thus a pE-neo vector was constructed. The pE-neo vector was digested with SfiI and BstXI to cut off a region of approximately 1 kbp containing a neomycin-resistance gene. A hygromycin gene was amplified by PCR reaction using pcDNA3.1/Hygro(+) (Invitrogen) as a template and using primer Hyg-Sfi5' (SEQ ID NO: 22) and primer Hyg-BstX3' (SEQ ID NO: 23). The amplified hygromycin gene was digested with SfiI and BstXI and inserted into the above pE-neo vector, thus a pE-hygr vector was constructed. The construction of the pE-neo vector and the pE-hygr vector was performed with reference to patent literature (JP 6279466 B).

### Construction of pE-IRES-GS-puro

An expression vector pPGKIH (Miyahara M. et. al., J. Biol. Chem. 275, 613-618 (2000)) was digested with restriction enzymes (XhoI and BamHI), and a DNA fragment containing an internal ribosome-entry site (IRES) derived from mouse encephalomyocarditis virus (EMCV), a hygromycin-resistance gene (Hyg^{r} gene), and a polyadenylated region (mPGKpA) of mouse phosphoglycerate kinase (mPGK) was excised. This DNA fragment was inserted between the XhoI and BamHI sites of pBluescript SK(-) (Stratagene), and this was designated as pBSK (IRES-Hygr-mPGKpA).

A DNA fragment containing a part of the IRES of EMCV was amplified by PCR using pBSK (IRES-Hygr-mPGKpA) as a template and using primer IRES5' (SEQ ID NO: 24) and primer IRES3' (SEQ ID NO: 25). This DNA fragment was digested with restriction enzymes (XhoI and HindIII) and inserted between the XhoI and HindIII sites of pBSK (IRES-Hygr-mPGKpA), and this was designated as pBSK (NotI-IRES-Hygr-mPGKpA). pBSK (NotI-IRES-Hygr-mPGKpA) was digested with restriction enzymes (NotI and BamHI) and inserted between the NotI and BamHI sites of the pE-hygr vector, and this was designated as plasmid pE-IRES-Hygr.

The expression vector pPGKIH was digested with EcoRI, and a DNA fragment composed of a base sequence containing the promoter region of mPGK (mPGKp) was excised. This DNA fragment was inserted into the EcoRI site of pBluescript SK(-) (Stratagene), and this was designated as mPGK promoter/pBS(-). A DNA fragment containing the promoter region of mPGK (mPGKp) was amplified by PCR using mPGK promoter/pBS(-) as a template and using primer mPGKP5' (SEQ ID NO: 26) and primer mPGKP3' (SEQ ID NO: 27). This DNA fragment was digested with restriction enzymes (BglII and EcoRI) and inserted between the BglII and EcoRI sites of pCI-neo (Promega), and this was designated as pPGK-neo. pE-IRES-Hygr was digested with restriction enzymes (NotI and BamHI), a DNA fragment (IRES-Hygr) was excised and inserted between the NotI and BamHI sites of pPGK-neo, and this was designated as pPGK-IRES-Hygr.

cDNA was prepared from CHO-K1 cells and used as a template, and a DNA fragment containing a GS gene was amplified by PCR using primer GS5' (SEQ ID NO: 28) and primer GS3' (SEQ ID NO: 29). This DNA fragment was digested with restriction enzymes (BalI and BamHI) and inserted between the BalI and BamHI sites of pPGK-IRES-Hygr, and this was designated as pPGK-IRES-GS-ΔpolyA.

A DNA fragment containing a puromycin-resistance gene (puro^{r} gene) was amplified by PCR using pCAGIPuro (Miyahara M. et. al., J. Biol. Chem. 275, 613-618 (2000)) as a template and using primer puro5' (SEQ ID NO: 30) and primer puro3' (SEQ ID NO: 31). This DNA fragment was inserted into pT7Blue T-Vector (Novagen), and this was designated as pT7 puro.
pT7 puro was digested with restriction enzymes (AflII and BstXI) and inserted between the AflII and BstXI sites of the expression vector pE-neo, and this was designated as pE-puro.

A DNA fragment containing the SV40 late polyadenylation region was amplified by PCR using pE-puro as a template and using primer SV40polyA5' (SEQ ID NO: 32) and primer SV40polyA3' (SEQ ID NO: 33). This DNA fragment was digested with restriction enzymes (NotI and HpaI) and inserted between the NotI and HpaI sites of the expression vector pE-puro, and this was designated as pE-puro (XhoI). pPGK-IRES-GS-ΔpolyA was digested with restriction enzymes (NotI and XhoI), a DNA fragment containing the IRES-GS region was excised, this was inserted between the NotI and XhoI sites of the expression vector pE-puro (XhoI), and this was designated as pE-IRES-GS-puro. The construction of pE-IRES-GS-puro was performed with reference to patent literature (JP 6279466 B).

### Construction of pE-mIRES-GS-puro(ΔE)

A region from the IRES to the GS of EMCV was amplified by PCR using the expression vector pE-IRES-GS-puro as a template and using primer mIRES-GS5' (SEQ ID NO: 34) and primer mIRES-GS3' (SEQ ID NO: 35), and a DNA fragment in which the initiation codon (ATG) located at the second position from the 5' side of the IRES of EMCV was mutated and destroyed was amplified. This DNA fragment and the primer IRES5' described above were used to amplify a DNA fragment containing the region from the IRES to the GS by PCR using the expression vector pE-IRES-GS-puro as a template. This DNA fragment was digested with restriction enzymes (NotI and PstI), the excised DNA fragment was inserted between the NotI and PstI sites of pBluescript SK(-) (Stratagene), and this was designated as mIRES/pBlueScript SK(-).

The expression vector pE-IRES-GS-puro was digested with SphI to cut off the SV40 enhancer region. The remaining DNA fragment was self-ligated, and this was designated as pE-IRES-GS-puro(ΔE). mIRES/pBlueScript SK(-) was digested with NotI and PstI, and a region containing the modified IRES (mIRES) and part of the GS gene was excised. Separately, pE-IRES-GS-puro(ΔE) was digested with NotI and PstI, the above region containing the mIRES and part of the GS gene was inserted into this, for constructing pE-mIRES-GS-puro(ΔE).

### Construction of pEM-hygr(LC3) and pE-mIRES-GSp-Fab-IDUA

A DNA fragment (CMVE-EF-1αp-IFNβMAR) containing the β-globin matrix attachment region (MAR), the CMV enhancer, the human EF-1α promoter, MluI and BamHI cleavage sites, and interferon β Mar was artificially synthesized (SEQ ID NO: 36). The HindIII sequence was introduced into the 5' side of this DNA fragment, and the EcoRI sequence was introduced into the 3' side. This DNA fragment was digested with HindIII and EcoRI and inserted between the HindIII and EcoRI sites of the pUC57 vector, and this was designated as JCR69 in pUC57. A DNA fragment (IRES-HygroR-mPGKpA) containing the MluI and BamHI cleavage sites, the IRES, the hygromycin-resistance gene, and an mPGK polyadenylation signal was artificially synthesized (SEQ ID NO: 40). This DNA fragment was inserted into the MluI and BamHI sites of the JCR69 in pUC57, and this was designated as pEM hygro.

A DNA fragment (SEQ ID NO: 37) containing a gene encoding the full-length of the light chain of the humanized anti-hTfR antibody with the amino acid sequence represented by SEQ ID NO: 14 was artificially synthesized and inserted into pUC57 Amp, and this was designated as JCR131 in pUC57 Amp. A MluI sequence was introduced into the 5' side of this DNA fragment, and a NotI sequence was introduced into the 3' side. This plasmid DNA was digested with MluI and NotI and inserted between MluI-NotI of the expression vector pEM hygro. The resulting vector was designated as pEM-hygr (LC3), which is a vector for expression of the light chain of the humanized anti-hTfR antibody.

A DNA fragment with a base sequence represented by SEQ ID NO: 39 containing a gene encoding a protein with an amino acid sequence represented by SEQ ID NO: 38 as a whole was artificially synthesized. In the DNA fragment, the human IDUA with the amino acid sequence represented by SEQ ID NO: 20 was linked to the C-terminal side of the Fab heavy chain of the humanized anti-hTfR antibody with the amino acid sequence represented by SEQ ID NO: 19 via the linker sequence represented by SEQ ID NO: 3. A MluI sequence was introduced into the 5' side of this DNA fragment, and a NotI sequence was introduced into the 3' side. This DNA fragment was digested with MluI and NotI and inserted between MluI and NotI of pE-mIRES-GS-puro(ΔE). The resulting vector was designated as pE-mIRES-GSp-Fab-IDUA, which is a vector for expression of a protein in which the hIDUA was linked to the C-terminal side of the Fab heavy chain of the humanized anti-hTfR antibody.

### Example 2: Preparation of cell line with high expression of humanized anti-hTfR antibody-hlDUA fusion protein

CHO cells (CHO-K1: obtained from American Type Culture Collection) were transformed with pEM-hygr(LC3) and pE-mIRES-GSp-Fab-IDUA constructed in Example 1 using NEPA21 (NEPAGENE) by the following method.

The transformation of the cells was generally performed in the following manner. CHO-K1 cells were suspended at a density of 2 × 10⁷ cells/mL in a 1:1 mixed solution of a CD OptiCHO (trade name) medium (Thermo Fisher Scientific Inc.) and PBS. Then, 50 µL of the cell suspension was collected and supplemented with 50 µL of a pEM-hygr(LC3) plasmid DNA solution diluted to 200 µg/mL with a 1:1 mixed solution of a CD OptiCHO (trade name) medium and PBS. The cells were electroporated using NEPA21 (NEPAGENE), and pEM-hygr (LC3) plasmid DNA was introduced into the cells. After overnight culture under conditions of 37°C and 5% CO₂, the cells were selectively cultured in a CD OptiCHO^{™} medium supplemented with 0.5 mg/mL hygromycin. pE-mIRES-GSp-Fab-IDUA plasmid DNA (linearized by digestion with AhdI) was introduced into the resulting cells in the same manner. After overnight culture under conditions of 37°C and 5% CO₂, the cells were selectively cultured in a CD OptiCHO (trade name) medium supplemented with 0.5 mg/mL hygromycin and 10 µg/mL puromycin. During the selective culture, the concentration of MSX was increased stepwise to a final MSX concentration of 300 µM, and cells showing drug resistance were selectively grown.

Then, the cells selected by the selective culture were seeded on a 96-well plate by limiting dilution to grow one cell or less per well and cultured for about 2 weeks for each cell to form a monoclonal colony. The culture supernatant of the well in which a monoclonal colony was formed was collected, the humanized antibody content was examined by ELISA, and a cell line with high expression of the humanized anti-hTfR antibody-hIDUA fusion protein was selected.

The ELISA at this time was generally performed in the following manner. To each well of a 96-well microtiter plate (Nunc), 100 µL each of a chicken anti-IDUA polyclonal antibody solution diluted to 5 µg/mL with a 0.05 M hydrogencarbonate buffer solution was added. The plate was allowed to stand at room temperature or 4°C for at least 1 hour, and the antibody was adsorbed to the plate. Then, after each well was washed three times with a tris-buffered saline (pH 8.0) supplemented with 0.05% Tween 20 (TBS-T), 300 µL of a tris-buffered saline (pH 8.0) supplemented with 1% BSA was added to each well, and the plate was allowed to stand at room temperature for 1 hour. Then, after each well was washed three times with TBS-T, 100 µL each of a culture supernatant or a purified humanized anti-hTfR antibody-hIDUA fusion protein product diluted to an appropriate concentration with a tris-buffered saline (pH 8.0) supplemented with 0.1% BSA and 0.05% Tween 20 (TBS-BT) was added to each well, and the plate was allowed to stand at room temperature for 1 hour. Then, the plate was washed three times with the TBS-BT, 50 µL each of an HRP-labeled anti-human IgG polyclonal antibody solution diluted with the TBS-BT was added to each well, and the plate was allowed to stand at room temperature for 1 hour. Each well was washed three times with the TBS-T, and a color was developed using an ELISA POD substrate TMB kit (Nakalai Tesque, Inc.). Then, 50 µL each of 1 mol/L sulfuric acid was added to each well to stop the reaction, and the absorbance at 450 nm was measured for each well using a 96-well plate reader. Cells corresponding to wells showing high measurements were selected as cells of a cell line with high expression of the humanized anti-hTfR antibody-hlDUA fusion protein. The cell line with high expression of the humanized anti-hTfR antibody-hIDUA fusion protein thus obtained was designated as a humanized anti-hTfR antibody-hIDUA expression line. The fusion protein of the humanized anti-hTfR antibody and hIDUA expressed by this cell line was used as a humanized anti-hTfR antibody-hIDUA fusion protein (humanized anti-hTfR antibody-hIDUA).

The resulting humanized anti-hTfR antibody-hIDUA expression line was suspended in a CD OptiCHO (trade name) medium containing 10 mg/L insulin, 16 µmol/L thymidine, 100 µmol/L hypoxanthine, 500 µg/mL hygromycin B, 10 µg/mL puromycin, 300 µmol/L MSX, and 10% (v/v) DMSO, then dispensed into cryotubes, and stored as seed cells in liquid nitrogen.

### Example 3: Culture of humanized anti-hTfR antibody-hlDUA expression line

To obtain humanized anti-hTfR antibody-hIDUA, the humanized anti-hTfR antibody-hIDUA expression line was cultured in the following manner. The humanized anti-hTfR antibody-hIDUA expression line obtained in Example 2 was suspended at a cell density of about 3 × 10⁵ cells/mL in about 170 L of a serum-free medium (CD OptiCHO^{™} medium, ThermoFisher SCIENTIFIC Inc.) containing 10 mg/L insulin, 16 µmol/L thymidine, and 100 µmol/L hypoxanthine and adjusted to pH 6.9. Then, 170 L of this cell suspension was transferred to a culture vessel. The medium was stirred with an impeller at a speed of approximately 100 rpm, the dissolved oxygen saturation of the medium was maintained at about 30%, and the cells were cultured in a temperature range from 34 to 37°C for about 10 days. During the culture period, cell density, cell viability, and glucose concentration and lactate concentration of the medium were monitored. When the glucose concentration of the medium became 3.0 g/L or less, a glucose solution was immediately added to the medium to give a glucose concentration of 3.5 g/L. During the culture period, a feed solution (EFFICIETFEED A+^{™}, ThermoFisher SCIENTIFIC Inc.) was added to the medium as appropriate. After completion of the culture, the medium was collected. The collected culture medium was filtered through Millistak+ HC Pod Filter grade D0HC (Merck & Co., Inc.) and further filtered through Millistak+ HCgrade X0HC (Merck & Co., Inc.), and a culture supernatant containing the humanized anti-hTfR antibody-hIDUA was obtained. This culture supernatant was ultrafiltered using Pellicon (trade name) 3 Cassette w/Ultracel PLCTK Membrane (pore size 30 kDa, membrane area 1.14 m², Merck & Co., Inc.), and concentrated until the liquid volume was about 1/14. This concentrated liquid was then filtered using Opticap XL600 (0.22 µm, Merck & Co., Inc.). The resulting liquid was used as a concentrated culture supernatant.

### Example 4: Purification of humanized anti-hTfR antibody-hlDUA

To the concentrated culture supernatant obtained in Example 3, 0.25-fold volume of a 2 M arginine solution (pH 7.0) were added. This solution was loaded onto a Capture Select (trade name) CH1-XL column (column volume of about 3.1 L, bed height of about 20 cm, Thermo Fisher Scientific Inc.) equilibrated with 4 column volume of a 25 mM MES buffer solution (pH 6.5) containing 400 mM arginine at a constant flow rate of 100 cm/hr, and the humanized anti-hTfR antibody-hIDUA was absorbed to the column. The Capture Select (trade name) CH1-XL column is an affinity column in which a ligand having a property of specifically binding to the CH1 domain of IgG antibodies is immobilized on the carrier.

The column was then washed by feeding 5 column volume of the same buffer solution at the same flow rate. Then, the column was further washed by feeding 3 column volume of a 25 mM MES buffer solution (pH 6.5) at the same flow rate. Then, the humanized anti-hTfR antibody-hIDUA adsorbed to the column was eluted with 5 column volume of a 10 mM sodium acetate-HCl buffer solution (pH 3.5). The eluate was received in a container charged with a 250 mM MES buffer solution (pH 6.0) in advance and was immediately neutralized.

To the eluate from the affinity column, a 250 mM MES buffer solution (pH 6.5) was added, and the pH of the eluate was adjusted to 6.0. This eluate was then filtered through an Opticap XL600 (pore size of 0.22 µm, Merck & Co., Inc.). This filtered solution was loaded onto a Capto adhere column (column volume of about 1.5 L, bed height of about 10 cm, GE Healthcare), which is a multimodal anion exchange column, equilibrated with 5 column volume of a 50 mM MES buffer solution (pH 6.0) containing 15 mM NaCl at a constant flow rate of 300 cm/hr. This loaded solution containing the humanized anti-hTfR antibody-hlDUA was collected. Capto adhere uses N-benzyl-N-methylethanolamine as a ligand and is a strong anion exchanger with selectivity based on electrostatic interaction, hydrogen bonding, hydrophobic interaction, and the like.

The column was then washed by feeding 5 column volume of the same buffer solution at the same flow rate, and this washing solution was collected.

The above loaded solution and washing solution were loaded onto a Capto MMC column (column volume of about 3.1 L, bed height of about 20 cm, GE Healthcare), which is a multimodal weak cation exchange column, equilibrated with 4 column volume of a 25 mM MES buffer solution (pH 6.5) containing 300 mM NaCl at a constant flow rate of 200 cm/hr. Capto MMC is a weak cation exchanger with selectivity based on hydrophobic interaction, hydrogen bond formation, and the like.

The column was then washed by feeding 5 column volume of the same buffer solution at the same flow rate. Then, the humanized anti-hTfR antibody-hIDUA adsorbed to the weak cation exchange column was eluted with 10 column volume of a 25 mM MES buffer solution (pH 6.5) containing 1 M NaCl.

To the eluate from the weak cation exchange column, 0.5-fold volume of 20 mM citrate buffer solution (pH 5.5) containing 0.8 mg/mL NaCl and 75 mg/mL sucrose was added, and the pH was adjusted to 5.8. Then, the solution was ultrafiltered using Pellicon (trade name) 3 Cassette w/Ultracel PLCTK Membrane (pore size of 30 kDa, membrane area of 0.57 m², Merck & Co., Inc.) and concentrated until the concentration of the humanized anti-hTfR antibody-hIDUA in the solution was about 30 mg/mL. This concentrated liquid was then filtered using Opticap XL600 (0.22 µm, Merck & Co., Inc.).

The above concentrated liquid was loaded onto a BioSEC column (column volume of about 9.4 L, bed height of 30 cm, Merck & Co., Inc.), which is a size exclusion column, equilibrated with 1.5 column volume of a 20 mM citrate buffer solution (pH 5.5) containing 0.8 mg/mL NaCl and 75 mg/mL sucrose, at a constant flow rate of 40 cm/hr, and the same buffer solution was further fed at the same flow rate. At this time, an absorptiometer for continuously measuring the absorbance of the eluate was placed in the flow path of the eluate from the size exclusion column to monitor the absorbance at 280 nm, and a fraction showing an absorption peak at 280 nm was collected as a fraction containing the humanized anti-hTfR antibody-hIDUA, and this was used as a purified product of the humanized anti-hTfR antibody-hIDUA.

### Example 5: Preparation of aqueous pharmaceutical composition

An aqueous pharmaceutical composition with the composition shown in Table 1 was prepared using the purified product of the humanized anti-hTfR antibody-hIDUA fusion protein obtained in Example 4. This aqueous pharmaceutical composition is filled and sealed in a glass or plastic vial, ampule, or syringe with a liquid volume of 1 to 10 mL, and stored at low temperatures (e.g., 4°C). A preparation obtained by filling and sealing this in a syringe is a prefilled syringe preparation.

**[Table 1]**

| Table 1 Composition of aqueous liquid preparation containing humanized anti-hTfR antibody-hlDUA | |
|---|---|
| Component | Composition |
| Humanized anti-hTfR antibody-hlDUA | 5 |
| Sodium chloride | 0.8 |
| Citric acid hydrate | 1.05 |
| Sodium citrate hydrate | 4.41 |
| Sucrose | 75 |
| Poloxamer 188 | 0.325 |
| Polysorbate 80 | 0.075 |
| pH | 5.5 |

### (The concentration of each excipient is indicated in mg/mL.)

### Example 6: Clinical trial (stage I)

To confirm the safety and efficacy of the aqueous pharmaceutical composition containing the humanized anti-hTfR antibody-hIDUA fusion protein prepared in Example 5 (hereinafter referred to as the "test drug") for mucopolysaccharidosis type I, a clinical trial (stage I) was performed.

In the clinical trial (stage I), subjects were patients with mucopolysaccharidosis type I (MPS I) meeting at least the inclusion criteria shown in Table 2. The number of subjects was 4. All the subjects had been receiving common enzyme replacement therapy for mucopolysaccharidosis type I before participating in the trial.

**[Table 2]**

| Table 2 Subject inclusion criteria |
|---|
| Inclusion criteria |
| Age is 18 years or older. |
| A person diagnosed with MPSI and meeting any of (1) to (3) below: |
| (1) The IDUA enzyme activity of leukocytes or cultured skin fibroblasts is less than 10% of the lower limit of the reference value, and an increase in urinary glycosaminoglycan (GAG) concentration with aging is observed (before enzyme replacement therapy). |
| (2) The IDUA enzyme activity of leukocytes or cultured skin fibroblasts is less than 10% of the lower limit of the reference value, and a mutation affecting the enzyme activity is present in both of IDUA genes on the homologous chromosomes. |
| (3) An increase in urinary GAG concentration with aging is observed (before enzyme replacement therapy), and a mutation affecting the enzyme activity is present in both of IDUA genes on the homologous chromosomes. |

The test drug was administered to the patient in the dosage and administration shown in Table 3. That is, the test drug was administered to each subject at a dose of 0.1 mg/kg body weight in a first administration, at a dose of 1.0 mg/kg body weight in a second administration, at a dose of 2.0 mg/kg body weight in a third administration, and at a dose of 4.0 mg/kg body weight in a fourth administration, a total of four times. Each dose was given at one-week intervals. In addition, each administration was conducted by intravenous drip infusion over about 3 hours while the condition of the subject was observed. In addition, after completion of the clinical trial, the patient was scheduled to receive common enzyme replacement therapy for mucopolysaccharidosis type I. In addition, cerebrospinal fluid was collected from each subject before the administration of the test drug and within 5 hours after the fourth administration. Furthermore, serum and urine were collected from each subject before the administration of the test drug, and one week after the fourth administration and before receiving common enzyme replacement therapy for mucopolysaccharidosis type I.

**[Table 3]**

| Table 3 Dosage and administration of test drug |
|---|
| Dosage and administration |
| The test drug is administered by intravenous drip infusion at a dose of 0.1 mg per kg body weight in a first administration. The test drug is administered by gradually increasing the dose to 1.0 mg/kg in a second administration, 2.0 mg/kg in a third administration, and 4.0 mg/kg in a fourth administration. The administration interval is one week. The total infusion volume is administered over about 3 hours. |

The efficacy of the test drug for mucopolysaccharidosis type I was evaluated by examining the items shown in Table 4. Table 4 shows some of the efficacy evaluation items described in the protocol for the clinical trial.

**[Table 4]**

| Table 4 Efficacy evaluation items of test drug (excerpts) |
|---|
| Efficacy evaluation items |
| Concentrations of heparan sulfate and dermatan sulfate in cerebrospinal fluid |
| Concentrations of heparan sulfate and dermatan sulfate in serum |
| Concentrations of heparan sulfate and dermatan sulfate in urine |
| Drug concentration in cerebrospinal fluid |
| Other clinical findings related to neurocognitive and behavioral changes |

The safety of the test drug for mucopolysaccharidosis type I was evaluated by examining the items shown in Table 5. Table 5 shows some of the safety evaluation items described in the protocol for the clinical trial.

**[Table 5]**

| Table 5 Safety evaluation items of test drug (excerpts) |
|---|
| Safety evaluation items |
| Adverse events |
| Clinical laboratory tests (hematology test, biochemical examination of blood, iron-related test, urinalysis) |
| Vital signs (pulse rate, body temperature, blood pressure) |
| 12-Lead electrocardiogram |
| Antibody tests (anti-IDUA antibodies, antibodies against humanized antibodies) |
| Infusion associated reaction (IAR) |

### Example 7: Efficacy evaluation of clinical trial (stage I)

### Concentrations of heparan sulfate and dermatan sulfate contained in cerebrospinal fluid

The measurement results of the concentrations of heparan sulfate and dermatan sulfate contained in the cerebrospinal fluids collected from each subject before the administration of the test drug collected from each subject and within 5 hours after the fourth administration in Example 6 are shown in FIGS. 1 and 2, respectively. For heparan sulfate, the average concentration of heparan sulfate contained in the cerebrospinal fluid of the subjects was 1132 ng/mL before the administration of the test drug, but the average concentration after the administration was significantly reduced to 467 ng/mL (FIG. 1). The average concentration of heparan sulfate contained in cerebrospinal fluids in non-mucopolysaccharidosis patients is approximately 360 ng/mL. Thus, further continual administration of the test drug was expected to be able to reduce the concentration of heparan sulfate contained in the cerebrospinal fluid to the level in non-mucopolysaccharidosis patients. The concentrations of heparan sulfate and dermatan sulfate were measured by the methods described in Examples 12 to 14.

For dermatan sulfate, the average concentration of dermatan sulfate contained in the cerebrospinal fluid of the subjects was 264 ng/mL before the administration of the test drug, but the average concentration after the administration was significantly reduced to 213 ng/mL (FIG. 2). Also for dermatan sulfate, similarly to heparan sulfate, further continual administration of the test drug was expected to be able to reduce the concentration of dermatan sulfate contained in the cerebrospinal fluid to the level in non-mucopolysaccharidosis patients.

These results indicate that the test drug administered to the patient by intravenous drip infusion crossed the BBB to reach the central nervous system and decomposed and removed the glycosaminoglycans accumulated therein, thus indicating that the test drug has an effect also on the abnormality in the central nervous system of mucopolysaccharidosis type I. In addition, from these results, the test drug is expected to exhibit its effect by administering once a week at a dose of 1 to 6 mg/kg body weight or 1 to 8 mg/kg body weight.

### Concentrations of heparan sulfate and dermatan sulfate contained in serum and urine

The measurement results of the concentrations of heparan sulfate and dermatan sulfate contained in the serums collected from each subject before the administration of the test drug collected from each subject, and one week after the fourth administration and before receiving common enzyme replacement therapy for mucopolysaccharidosis type I in Example 6 are shown in FIGS. 3 and 4, respectively. For heparan sulfate, the average concentration of heparan sulfate contained in the serum of the subjects was 359 ng/mL before the administration of the test drug, but the average concentration after the administration was significantly reduced to 217 ng/mL (FIG. 3). For dermatan sulfate, the average concentration of dermatan sulfate contained in the serum of the subjects was 965 ng/mL before the administration of the test drug, but the average concentration after the administration was significantly reduced to 867 ng/mL (FIG. 4).

The measurement results of the concentrations of heparan sulfate and dermatan sulfate contained in the urines collected from each subject before the administration of the test drug, and one week after the fourth administration and before receiving common enzyme replacement therapy for mucopolysaccharidosis type I in Example 6 are shown in FIGS. 5 and 6, respectively. For heparan sulfate, the average concentration of heparan sulfate contained in the urine of the subjects was 31.0 µg/mg creatinine before the administration of the test drug, but the average concentration after the administration was significantly reduced to 16.0 µg/mg creatinine (FIG. 5). For dermatan sulfate, the average concentration of dermatan sulfate contained in the urine of the subjects was 22.7 µg/mg creatinine before the administration of the test drug, but the average concentration after the administration was significantly reduced to 17.1 µg/mg creatinine (FIG. 6). These results indicate that the test drug administered to the patient by intravenous drip infusion decomposed and removed the glycosaminoglycans accumulated not only in the central nervous system but also in the tissues throughout the body, and indicate that the test drug has an effect also on the abnormalities in the central nervous system and other tissues of mucopolysaccharidosis type I. In addition, in a patient in whom the glycosaminoglycans accumulated in the central nervous system and other tissues have been decomposed and removed, amelioration of functional disorders is expected, such as enabling conversation for a longer period; enabling writing more letters and/or characters; increasing vitality; reducing joint pain in the lower back, knees, and the like; reducing pain and muscle and joint stiffness associated with walking; making it easier to move fingertips, such as opening a can, and perform whole body exercise, such as basketball; and improving language skills.

### Example 8: Safety evaluation of clinical trial (stage I)

No serious adverse events were observed in all the subjects during the administration period of the test drug. That is, this indicates that the test drug can be safely administered once a week at a dose of 0.1 to 4 mg/kg body weight.

### Example 9: Clinical trial (stage II)

A clinical trial (stage II) was to confirm the safety and efficacy for mucopolysaccharidosis type I when the test drug was administered for 3 months.

In the clinical trial (stage II), subjects were patients with mucopolysaccharidosis type I (MPS I) meeting at least the inclusion criteria shown in Table 2. All the subjects had been receiving common enzyme replacement therapy for mucopolysaccharidosis type I before participating in the trial.

The clinical trial (stage II) was composed of two trials. In a clinical trial (stage II) first trial, the test drug was administered to the patient in the dosage and administration shown in Table 6. That is, the test drug was administered to each subject at a dose of 1.0 mg/kg body weight in a first administration, at a dose of 2.0 mg/kg body weight in a second administration, and at a dose of 4.0 mg/kg body weight 10 times from third to 12th administrations. Each dose was given at one-week intervals. In addition, each administration was conducted by intravenous drip infusion over about 3 hours while the condition of the subject was observed. The number of subjects is 2.

**[Table 6]**

| Table 6 Dosage and administration of test drug (clinical trial (stage II) first trial) |
|---|
| Dosage and administration |
| The test drug is administered by intravenous drip infusion at a dose of 1.0 mg per kg body weight in a first administration. The test drug is administered by intravenous drip infusion at a dose of 2.0 mg/kg in a second administration and then at a dose of 4.0 mg/kg a total of 10 times from third to 12th administrations. The administration interval is one week. The total infusion volume is administered over about 3 hours. |

In a clinical trial (stage II) second trial, the test drug was administered to the patient in the dosage and administration shown in Table 7. That is, the subjects were divided into two groups, and the test drug was administered to one group at a dose of 2.0 mg/kg body weight (2.0 mg/kg body weight administration group) and to the other group at a dose of 4.0 mg/kg body weight (4.0 mg/kg body weight administration group). In each group, the test drug was administered 12 times at one-week intervals. In addition, each administration was conducted by intravenous drip infusion over about 3 hours while the condition of the subject was observed. The trial was conducted in an unblinded manner with 15 subjects.

**[Table 7]**

| Table 7 Dosage and administration of test drug (clinical trial (stage II) second trial) |
|---|
| Dosage and administration |
| The subjects are divided into two groups, and the test drug is administered to one group at a dose of 2.0 mg/kg body weight (2.0 mg/kg body weight administration group) and to the other group at a dose of 4.0 mg/kg body weight (4.0 mg/kg body weight administration group). |

In both the clinical trial (stage II) first and second trials, cerebrospinal fluid was collected from each subject before the administration of the test drug and within 5 hours after the 12th administration. Furthermore, serum and urine were collected from each subject before the administration of the test drug, and one week after the 12th administration and before receiving common enzyme replacement therapy for mucopolysaccharidosis type I. In the second trial, cerebrospinal fluid, serum, and urine were collected only from 6 subjects who completed the administration among the 15 subjects scheduled to participate the trial.

In addition to examining the items shown in Table 4, the efficacy of the test drug for mucopolysaccharidosis type I was also evaluated by assigning the subjects to the Brief Visuospatial Memory Test-Revised (BVMT-R), Hopkins Verbal Language Learning Test-Revised (HVLT-R), and Test of Variable of Attention (TOVA).

The safety of the test drug for mucopolysaccharidosis type I was evaluated by examining the items shown in Table 5. Table 5 shows some of the safety evaluation items described in the protocol for the clinical trial.

### Example 10: Efficacy evaluation of clinical trial (stage II)

### Concentrations of heparan sulfate and dermatan sulfate contained in cerebrospinal fluid

The measurement results of the concentrations of heparan sulfate and dermatan sulfate contained in the cerebrospinal fluids collected from each subject in the first trial before the administration of the test drug and within 5 hours after the 12th administration in Example 9 are shown in FIGS. 7 and 8, respectively. For heparan sulfate, the concentration of heparan sulfate contained in the cerebrospinal fluid of the subject was reduced to 1/3 or less in all the subjects after the administration of the test drug compared with before the administration (FIG. 7). In addition, the concentration of heparan sulfate contained in the cerebrospinal fluid of the subject was 1400 ng/mL or less in all the subjects and dropped below the average concentration of heparan sulfate contained in cerebrospinal fluids of non-mucopolysaccharidosis patients of about 360 ng/mL. From these results, further continual administration of the test drug was expected to be able to reduce the concentration of heparan sulfate contained in the cerebrospinal fluid to the level in non-mucopolysaccharidosis patients and maintain this.

For dermatan sulfate, the concentration of dermatan sulfate contained in the cerebrospinal fluid of the subject was reduced in all the subjects after the administration of the test drug compared with before the administration (FIG. 8). The average concentration of dermatan sulfate contained in cerebrospinal fluids in non-mucopolysaccharidosis patients is about 220 ng/mL. Also for dermatan sulfate, similarly to heparan sulfate, further continual administration of the test drug was expected to be able to reduce the concentration of dermatan sulfate contained in the cerebrospinal fluid to the level in non-mucopolysaccharidosis patients and maintain this.

The measurement results of the concentrations of heparan sulfate and dermatan sulfate contained in the cerebrospinal fluids collected from each subject in the second trial before the administration of the test drug and within 5 hours after the 12th administration in Example 9 are shown in FIGS. 9 and 10, respectively. For heparan sulfate, the concentration of heparan sulfate contained in the cerebrospinal fluid of the subject was reduced to 1/3 or less in all the subjects after the administration of the test drug compared with before the administration (FIG. 9). In addition, the concentration of heparan sulfate contained in the cerebrospinal fluid of the subject was 1400 ng/mL or less in all the subjects and neared or dropped below the average concentrations of heparan sulfate contained in cerebrospinal fluids of non-mucopolysaccharidosis patients of about 360 ng/mL. Further continual administration of the test drug was expected to be able to reduce the concentration of heparan sulfate contained in the cerebrospinal fluid to the level in non-mucopolysaccharidosis patients and maintain this.

For dermatan sulfate, the concentration of dermatan sulfate contained in the cerebrospinal fluid of the subject was reduced in all the subjects after the administration of the test drug compared with before the administration (FIG. 10). The average concentration of dermatan sulfate contained in cerebrospinal fluids in non-mucopolysaccharidosis patients is about 220 ng/mL. Also for dermatan sulfate, similarly to heparan sulfate, further continual administration of the test drug was expected to be able to reduce the concentration of dermatan sulfate contained in the cerebrospinal fluid to the level in non-mucopolysaccharidosis patients and maintain this. The concentrations of heparan sulfate and dermatan sulfate were measured by the methods described in Examples 12 to 14.

These results indicate that the test drug administered to the patient by intravenous drip infusion crossed the BBB to reach the central nervous system and decomposed and removed the glycosaminoglycans accumulated therein, thus indicating that the test drug has an effect also on the abnormality in the central nervous system of mucopolysaccharidosis type I. In addition, from these results, the test drug is expected to exhibit its effect by administering once a week at a dose of 1 to 6 mg/kg body weight or 1 to 8 mg/kg body weight.

### Concentrations of heparan sulfate and dermatan sulfate contained in serum

The measurement results of the concentrations of heparan sulfate and dermatan sulfate contained in the serums collected from each subject in the first trial before the administration of the test drug, and within one week after the 12th administration and before receiving common enzyme replacement therapy for mucopolysaccharidosis type I in Example 9 are shown in FIGS. 11 and 12, respectively. The results confirmed that the concentrations of both heparan sulfate and dermatan sulfate tended to generally decrease after the administration of the test drug compared with before the administration (FIGS. 11 and 12).

The measurement results of the concentrations of heparan sulfate and dermatan sulfate contained in the serums each collected from each subject in the second trial before the administration of the test drug, and within one week after the 12th administration and before receiving common enzyme replacement therapy for mucopolysaccharidosis type I in Example 9 are shown in FIGS. 13 and 14, respectively. The results confirmed that the concentrations of both heparan sulfate and dermatan sulfate tended to generally decrease after the administration of the test drug compared with before the administration (FIGS. 13 and 14).

The above results indicate that the test drug administered to the patient by intravenous drip infusion decomposed and removed the glycosaminoglycans accumulated not only in the central nervous system but also in the tissues throughout the body, and indicate that the test drug has an effect on the abnormalities in the central nervous system and other tissues of mucopolysaccharidosis type I too. In addition, in a patient in whom the glycosaminoglycans accumulated in the central nervous system and other tissues have been decomposed and removed, amelioration of functional disorders is expected to produce an effect, such as ability to have a longer conversation; ability to write more letters and/or characters; an increase in vitality; reduction in joint pain in the lower back, knees, and the like; reduction of pain and muscle and joint stiffness associated with walking; improved mobility of fingers, such as opening a can, and ability to perform whole body exercise, such as basketball; and improvement in language skills.

### Example 11: Safety evaluation of clinical trial (stage II)

No serious adverse events were observed in all the subjects during the administration period of the test drug. That is, this indicates that the test drug can be safely administered once a week at a dose of 0.1 mg/kg to 4 mg/kg body weight.

### Example 12: Method for measuring heparan sulfate and dermatan sulfate (preparation of various solutions)

Solutions (a) to (k) used in the test were prepared by the following procedure.
(a) MeCN/water:
   0.5 mL of water for injection and 4.5 mL of acetonitrile were mixed, and this was used as an MeCN/water. This solution was prepared at the time of use.
(b) Deuterium-labeled solvent:
   In an ice bath, 240 µL of acetyl chloride was added dropwise to 1.5 mL of methanol-d₄ (Sigma-Aldrich), and this was used as a deuterium-labeled solvent. This solution was prepared at the time of use.
(c) PBS/citrate solution:
   Citric acid was dissolved in pure water to prepare a citric acid solution with a concentration of 10 mM. Furthermore, trisodium citrate dihydrate was dissolved in pure water to prepare a sodium citrate solution with a concentration of 10 mM. The sodium citrate solution was added dropwise to the citric acid solution to adjust the pH to 3.0. This solution was used as a 10 mM citrate buffer solution (pH 3.0). In addition, a solution obtained by adding 2 mL of PBS to 18 mL of the 10 mM citrate buffer solution (pH 3.0) was used as a PBS/citrate solution.
(d) Mobile phase A:
   A mixture obtained by adding and mixing 2.5 mL of a 1 M ammonium formate aqueous solution and 400 µL of an ammonium hydroxide aqueous solution (25% NH₄OH) to 247.5 mL of pure water was used as a mobile phase A. This solution was prepared at the time of use.
(e) Mobile phase B:
   A mixture obtained by mixing 5 mL of a 1 M ammonium formate aqueous solution, 450 mL of acetonitrile, and 800 µL of an ammonium hydroxide aqueous solution (25% NH₄OH) to 45 mL of pure water was used as a mobile phase B. This solution was prepared at the time of use.
(f) Heparan sulfate standard stock solution (HS standard stock solution):
   Heparan sulfate (Iduron Ltd) was weighed in a 1.5-mL microtube and dissolved by adding water for injection to prepare a solution with a concentration of 5.0 mg/mL. Into 0.5-mL screw cap tubes, 15-µL aliquots of the prepared solution were dispensed and stored frozen (-15°C or lower) until use. This solution was used as an HS standard stock solution.
(g) Dermatan sulfate standard stock solution (DS standard stock solution):
   Chondroitin sulfate B sodium salt from porcine intestinal mucosa (Sigma-Aldrich) was weighed in a 1.5-mL microtube and dissolved by adding water for injection to prepare a solution with a concentration of 5.0 mg/mL. Into 0.5-mL screw cap tubes, 15-µL aliquots of the prepared solution were dispensed and stored frozen (-15°C or lower) until use. This solution was used as a DS standard stock solution.
(h) Dermatan sulfate internal standard solution (DS internal standard solution):
   Into a borosilicate screw-top test tube, 40 µL of the DS standard stock solution was pipetted, and the solvent was distilled off under a nitrogen stream. To the dried product, 400 µL of the deuterium-labeled solution was added, and the mixture was stirred and then reacted at 65°C for 75 minutes to perform deuteriomethanolysis. After the reaction, the solvent was distilled off under a nitrogen stream. To the dried product, 500 µL of the MeCN/water was added, and the mixture was ultrasonicated for 30 minutes. Into 0.5-mL screw cap tubes, 20-µL aliquots of the prepared solution were dispensed and stored frozen (-15°C or lower) until use. This solution was used as a dermatan sulfate internal standard solution (DS internal standard solution).
(i) Heparan sulfate internal standard solution (HS internal standard solution):
   Into a borosilicate screw-top test tube, 40 µL of the HS standard stock solution was pipetted, and the solvent was distilled off under a nitrogen stream. To the dried product, 400 µL of the deuterium-labeled solution was added, and the mixture was stirred and then reacted at 65°C for 75 minutes to perform deuteriomethanolysis. After the reaction, the solvent was distilled off under a nitrogen stream. To the dried product, 500 µL of the MeCN/water was added, and the mixture was ultrasonicated for 30 minutes. Into 0.5-mL screw cap tubes, 20-µL aliquots of the prepared solution were dispensed and stored frozen (-15°C or lower) until use. This solution was used as a heparan sulfate internal standard solution (HS internal standard solution).
(j) Solution for sample dissolution:
   To 5 mL of the MeCN/water, 1 µL of the HS internal standard solution and 1 µL of the DS internal standard solution were added, and the mixture was stirred and then ultrasonicated for 30 minutes. This solution was used as a solution for sample dissolution. This solution was prepared at the time of use.
(k) Solutions for calibration curve preparation:
   480 µL of the PBS/citrate solution was pipetted, and 10 µL each of the HS standard stock solution and the DS standard stock solution was added to this to prepare a solution containing 100 µg/mL each of dermatan sulfate and heparan sulfate. This solution was diluted with the PBS/citrate solution to prepare a solution containing 2500 ng/mL each of dermatan sulfate and heparan sulfate. This solution was serially diluted in two stages with the PBS/citrate solution to prepare solutions containing dermatan sulfate and heparan sulfate each in a concentration of 25 to 2500 ng/mL. This solution was used as a solution for calibration curve preparation.

### Example 13: Method for measuring heparan sulfate and dermatan sulfate (methanolysis reaction)

20 µL each of the solutions for calibration curve preparation prepared in Example 12 above were pipetted and individually dispensed into borosilicate screw-top test tubes. In addition, the PBS/citrate solution was dispensed into borosilicate screw-top test tubes as a blank. The solvent in the test tube was distilled off under a nitrogen stream (N = 1). To the dried product, 20 µL of 2,2-dimethoxypropane and 200 µL of methanol hydrochloride with a hydrochloride concentration of 3N were added, and the mixture was stirred and then subjected to a methanolysis reaction at 70°C for 90 minutes using a constant-temperature water bath. After the reaction, the solvent was distilled off under a nitrogen stream. The dried product was dissolved by adding 50 µL of the solution for sample dissolution, the solution was then centrifuged (15000 rpm, 10 minutes, room temperature), and the supernatant was collected in a vial.

In addition, the dermatan sulfate standard stock solution and the heparan sulfate standard stock solution prepared in Example 12 were diluted with the PBS/citrate solution to prepare four quality control samples (QC samples) containing the dermatan sulfate standard stock solution and the heparan sulfate standard stock solution both in concentrations of 25.0 ng, 50.0 ng/mL, 500 ng/mL, and 2000 ng/mL as QC-LL, QC-L, QC-M, and QC-H, respectively. 20 µL each of these were pipetted and individually dispensed into borosilicate screw-cap test tubes, and the solvent in the test tubes was distilled off under a nitrogen stream. To the dried product, 20 µL of 2,2-dimethoxypropane and 200 µL of methanol hydrochloride with a hydrochloride concentration of 3N were added, and the mixture was stirred and then subjected to a methanolysis reaction at 70°C for 90 minutes using a constant-temperature water bath. After the reaction, the solvent was distilled off under a nitrogen stream. The dried product was dissolved by adding 50 µL of the solution for sample dissolution, the solution was then centrifuged (15000 rpm, 10 minutes, room temperature), and the supernatant was collected in a vial.

In addition, the cerebrospinal fluid, serum, and urine collected in Example 6 or 9 were each dispensed into borosilicate screw-top test tubes, and the solvent in the test tubes was distilled off under a nitrogen stream. To the dried product, 20 µL of 2,2-dimethoxypropane and 200 µL of methanol hydrochloride with a hydrochloride concentration of 3N were added, and the mixture was stirred and then subjected to a methanolysis reaction at 70°C for 90 minutes using a constant-temperature water bath. After the reaction, the solvent was distilled off under a nitrogen stream. The dried product was dissolved by adding 50 µL of the solution for sample dissolution, the solution was then centrifuged (15000 rpm, 10 minutes, room temperature), and the supernatant was collected in a vial.

### Example 14: Method for measuring heparan sulfate and dermatan sulfate (LC/MS/MS analysis)

LC/MS/MS analysis was performed using a combination of hydrophilic interaction ultra-high performance liquid chromatography and a tandem quadrupole mass spectrometer. QTRAP5500 (AB Sciex) was used as a mass spectrometer (MS/MS instrument), and Nexera X2 (Shimadzu Corporation) was set as an HPLC instrument to this. In addition, Acquity UPLC (trade name) BEH Amid 1.7 µm (2.1 × 50 mm, Waters Corporation) was used as an LC column. The mobile phases A and B prepared in Example 12 were used as mobile phases. Furthermore, the column temperature was set at 50°C.

After the column was equilibrated with a mixed solution composed of 6% (v/v) of the mobile phase A and 94% (v/v) of the mobile phase B, 10 µL of a sample was injected, and the chromatography was performed under the gradient conditions of the mobile phases shown in Table 8. The flow rate of the mobile phases was set at 0.4 mL/min.

**[Table 8]**

| Table 8 Conditions of liquid chromatography | | |
|---|---|---|
| Elapsed time after sample injection (min) | Mobile phase A (%(v/v)) | Mobile phase B (%(v/v)) |
| 0 | 6 | 94 |
| 1 | 6 | 94 |
| 4 | 30 | 70 |
| 4.01 | 6 | 94 |
| 6 | 6 | 94 |

The ion source parameters of the MS/MS instrument were set according to the instruction manual of QTRAP5500 (AB Sciex) as shown in Table 9.

**[Table 9]**

| Table 9 Setting of various parameters of ion source of MS/MS instrument | |
|---|---|
| Ion source | ESI (Turbo V) |
| Polarity | Positive |
| Scan type | MRM |
| IonSpray voltage | 5500 V |
| Heater gas temperature | 500°C |
| Curtain gas (CUR) | 30.0 psi |
| Collision gas (CAD) | 8 psi |
| Ion source gas 1 (GS1) | 70.0 psi |
| Ion source gas 2 (GS2) | 70.0 psi |
| Entrance potential | 10.0 V |
| Duration | 4.00 min |

Areas of peaks (detection peaks) detected on a chromatographic chart of product ions derived from dermatan sulfate and heparan sulfate contained in each solution for calibration curve preparation were determined by measuring the solutions for calibration curve preparation and the QC samples. In addition, areas of detection peaks of product ions derived from the DS internal standard solution and the HS internal standard solution were determined. The solutions for calibration curve preparation were each measured once (N = 1), and the QC samples (QC-LL, QC-L, QC-M, and QC-H) were each measured three times (N = 3).

Dermatan sulfate and heparan sulfate contained in the DS internal standard solution and the HS internal standard solution were labeled with deuterium. Thus, mass-to-charge ratios (m/z) of precursor ions derived from these were 432 and 390, respectively, which were larger than those of precursor ions derived from unlabeled dermatan sulfate and heparan sulfate. Mass-to-charge ratios (m/z) of precursor ions derived from unlabeled dermatan sulfate and heparan sulfate were 426 and 384, respectively. Thus, these precursor ions were separated based on the mass-to-charge ratios (m/z) of the ions in the quadrupole (Q1) and thus detectable individually. The (m/z) of the precursor ions and the product ions are summarized and shown in Table 10.

**[Table 10]**

| Table 10 (m/z) of precursor ions and product ions | | |
|---|---|---|
| | Precursor ions (m/z) | Productions (m/z) |
| Dermatan sulfate | 426 | 236 |
| Dermatan sulfate (deuterium-labeled) | 432 | 239 |
| Heparan sulfate | 384 | 162 |
| Heparan sulfate (deuterium-labeled) | 390 | 162 |

The area ratio (DS detection peak area/DS-IS detection peak area) of the area of the detection peak derived from the DS internal standard solution (DS-IS detection peak area) to the area of the detection peak derived from dermatan sulfate contained in each solution for calibration curve preparation (DS detection peak area) was determined. A calibration curve was prepared by plotting this value on the vertical axis, plotting the concentration of dermatan sulfate in each solution for calibration curve preparation on the horizontal axis, and calculating a regression equation using quadratic programming.

In addition, the area ratio (HS detection peak area/HS-IS detection peak area) of the area of the detection peak derived from the HS internal standard solution (HS-IS detection peak area) to the area of the detection peak derived from heparan sulfate contained in each solution for calibration curve preparation (HS detection peak area) was determined. A calibration curve was prepared by plotting this value on the vertical axis, plotting the concentration of heparan sulfate in each solution for calibration curve preparation on the horizontal axis, and calculating a regression equation using quadratic programming.

The measurements of cerebrospinal fluid, serum, and urine of each subject were interpolated into the above calibration curve, and the concentrations of DS and HS contained in each sample were determined. The measurements of DS and HS in urine were corrected for creatinine, and the amounts contained in urine containing 1 mg of creatinine (µg/mg creatinine) were calculated.

### Industrial Applicability

According to the present invention, there can be provided a new drug that can be used, for example, in enzyme replacement therapy for mucopolysaccharidosis type I patients.

### Sequence table free text

SEQ ID NO: 1: amino acid sequence of linker example 1
SEQ ID NO: 2: amino acid sequence of linker example 2
SEQ ID NO: 3: amino acid sequence of linker example 3
SEQ ID NO: 4: amino acid sequence 1 of light chain CDR1
SEQ ID NO: 5: amino acid sequence 2 of light chain CDR1
SEQ ID NO 6: amino acid sequence 1 of light chain CDR2
SEQ ID NO: 7: amino acid sequence 2 of light chain CDR2
SEQ ID NO: 8: amino acid sequence 1 of light chain CDR3
SEQ ID NO: 9: amino acid sequence 1 of heavy chain CDR1
SEQ ID NO: 10: amino acid sequence 2 of heavy chain CDR1
SEQ ID NO: 11: amino acid sequence 1 of heavy chain CDR2
SEQ ID NO: 12: amino acid sequence 2 of heavy chain CDR2
SEQ ID NO: 13: amino acid sequence 1 of heavy chain CDR3
SEQ ID NO: 14: amino acid sequence 2 of heavy chain CDR3
SEQ ID NO: 15: amino acid sequence of heavy chain framework region 3
SEQ ID NO: 16: amino acid sequence of variable region of heavy chain
SEQ ID NO 17: amino acid sequence of variable region of light chain
SEQ ID NO: 18: amino acid sequence of light chain
SEQ ID NO 19: amino acid sequence of Fab heavy chain
SEQ ID NO: 20: amino acid sequence 1 of human IDUA
SEQ ID NO: 21: amino acid sequence 2 of human IDUA
SEQ ID NO: 22: primer Hyg-Sfi5', synthetic sequence
SEQ ID NO: 23: primer Hyg-BstX3', synthetic sequence
SEQ ID NO: 24: primer IRES5', synthetic sequence
SEQ ID NO: 25: primer IRES3', synthetic sequence
SEQ ID NO: 26: primer mPGKP5', synthetic sequence
SEQ ID NO: 27: primer mPGKP3', synthetic sequence
SEQ ID NO: 28: primer GS5', synthetic sequence
SEQ ID NO: 29: primer GS3', synthetic sequence
SEQ ID NO: 30: primer puro5', synthetic sequence
SEQ ID NO: 31: primer puro3', synthetic sequence
SEQ ID NO: 32: primer SV40polyA5', synthetic sequence
SEQ ID NO 33: primer SV40polyA3', synthetic sequence
SEQ ID NO: 34: primer mIRES-GS5', synthetic sequence
SEQ ID NO: 35: primer mIRES-GS3', synthetic sequence
SEQ ID NO 36: CMVE-EF-1αp-IFNβMAR, synthetic sequence
SEQ ID NO: 37: base sequence encoding amino acid sequence of light chain, synthetic sequence
SEQ ID NO 38: amino acid sequence of fusion protein of Fab heavy chain and human IDUA
SEQ ID NO: 39: base sequence containing gene encoding amino acid sequence of fusion protein of Fab heavy chain and human IDUA, synthetic sequence
SEQ ID NO: 40: IRES-HygroR-mPGKpA, synthetic sequence

## Claims

1. A pharmaceutical composition, comprising, as an active ingredient, a fusion protein of an anti-human transferrin receptor antibody and human α-L-iduronidase, wherein the fusion protein is administered to a patient with mucopolysaccharidosis type I by intravenous infusion at a dose of 0.1 to 10 mg/kg body weight.

2. The pharmaceutical composition according to claim 1, wherein the fusion protein is administered at a dose of 0.1 to 8 mg/kg body weight.

3. The pharmaceutical composition according to claim 1, wherein the fusion protein is administered at a dose of 1 to 6 mg/kg body weight.

4. The pharmaceutical composition according to claim 1, wherein the fusion protein is administered at a dose of 2 mg/kg body weight or 4 mg/kg body weight.

5. The pharmaceutical composition according to any one of claims 1 to 4, wherein the fusion protein is administered at a rate of 0.33 mg/hour to 200 mg/hour.

6. The pharmaceutical composition according to any one of claims 1 to 4, wherein the fusion protein is administered over a period of 3 hours.

7. The pharmaceutical composition according to any one of claims 1 to 6, wherein the fusion protein is administered by intravenous drip.

8. The pharmaceutical composition according to any one of claims 1 to 7, wherein the administration is performed continually for at least 3 months at intervals of 5 days to 21 days.

9. The pharmaceutical composition according to any one of claims 1 to 7, wherein the administration is performed continually for at least 1 month at intervals of 7 days.

10. The pharmaceutical composition according to claim 8 or 9, wherein the fusion protein is administered at a dose of 0.1 to 2 mg/kg body weight at a first administration and is administered at an increased dose at second and subsequent administrations.

11. The pharmaceutical composition according to claim 8 or 9, wherein the fusion protein is administered at a dose of 0.1 to 2 mg/kg body weight at a first administration and is then administered at a maintenance dose of 2 to 6 mg/kg body weight.

12. The pharmaceutical composition according to claim 8 or 10, wherein the administration is performed at a maintenance dose of 2 mg/kg body weight or 4 mg/kg body weight.

13. The pharmaceutical composition according to any one of claims 1 to 12, wherein the anti-human transferrin receptor antibody is a Fab.

14. The pharmaceutical composition according to any one of claims 1 to 13, wherein the human α-L-iduronidase is linked directly or via a linker to a light chain of the anti-human transferrin receptor antibody at the C-terminal side or the N-terminal side, or to a heavy chain of the anti-human transferrin receptor antibody at the C-terminal side or the N-terminal side.

15. The pharmaceutical composition according to any one of claims 1 to 13, wherein the human α-L-iduronidase is linked via a linker to the heavy chain of the anti-human transferrin receptor antibody at the C-terminal side.

16. The pharmaceutical composition according to claim 14 or 15, wherein the linker is a peptide consisting of 1 to 150 amino acid residues.

17. The pharmaceutical composition according to claim 16, wherein the linker is a peptide comprising the amino acid sequence selected from the group consisting of one glycine, one serine, the amino acid sequence Gly-Ser, the amino acid sequence Gly-Gly-Ser, the amino acid sequence of SEQ ID NO: 1, the amino acid sequence of SEQ ID NO: 2, the amino acid sequence of SEQ ID NO: 3, and the amino acid sequence consisting of 1 to 10 of the aforementioned amino acid sequences that are consecutively linked.

18. The pharmaceutical composition according to claim 16, wherein the linker is a peptide consisting of the amino acid sequence of SEQ ID NO: 3.

19. The pharmaceutical composition according to any one of claims 1 to 18, wherein
the anti-human transferrin receptor antibody comprises, in a variable region of the light chain, the amino acid sequence of SEQ ID NO: 4 or SEQ ID NO: 5 as CDR1, the amino acid sequence of SEQ ID NO: 6 or SEQ ID NO: 7 or the amino acid sequence Lys-Val-Ser as CDR2, and the amino acid sequence of SEQ ID NO: 8 as CDR3, and comprises, in a variable region of the heavy chain, the amino acid sequence of SEQ ID NO: 9 or 10 as CDR1, the amino acid sequence of SEQ ID NO: 11 or 2 as CDR2, and the amino acid sequence of SEQ ID NO: 13 or 14 as CDR3; and
the human α-L-iduronidase is linked to the light chain of the anti-human transferrin receptor antibody at the C-terminal side or the N-terminal side, or to the heavy chain of the anti-human transferrin receptor antibody at the C-terminal side or the N-terminal side.

20. The pharmaceutical composition according to claim 19, wherein the variable region of the heavy chain comprises the amino acid sequence of SEQ ID NO: 16.

21. The pharmaceutical composition according to claim 20, wherein the heavy chain is a Fab heavy chain, and the Fab heavy chain comprises the amino acid sequence of SEQ ID NO: 19.

22. The pharmaceutical composition according to any one of claims 19 to 21, wherein the variable region of the light chain comprises the amino acid sequence of SEQ ID NO: 17.

23. The pharmaceutical composition according to claim 22, wherein the light chain comprises the amino acid sequence of SEQ ID NO: 18.

24. The pharmaceutical composition according to any one of claims 1 to 23, wherein the human α-L-iduronidase comprises the amino acid sequence having at least 85% identity with the amino acid sequence of SEQ ID NO: 20 or the amino acid sequence of SEQ ID NO: 21.

25. The pharmaceutical composition according to any one of claims 1 to 23, wherein the human α-L-iduronidase comprises the amino acid sequence of SEQ ID NO: 20 or the amino acid sequence of SEQ ID NO: 21.

26. The pharmaceutical composition according to claim 19, wherein
the light chain of the anti-human transferrin receptor antibody comprises the amino acid sequence of SEQ ID NO: 18,
the heavy chain of the anti-human transferrin receptor antibody comprises the amino acid sequence of SEQ ID NO: 19, and
the heavy chain is linked at the C-terminal side of the heavy chain to the human α-L-iduronidase with the amino acid sequence of SEQ ID NO: 20 or SEQ ID NO: 21 via the linker of the amino acid sequence of SEQ ID NO: 3.

27. The pharmaceutical composition according to claim 19, wherein
the light chain of the anti-human transferrin receptor antibody comprises the amino acid sequence of SEQ ID NO: 18,
the heavy chain of the anti-human transferrin receptor antibody comprises the amino acid sequence of SEQ ID NO: 19, and
the heavy chain is linked at the C-terminal side of the heavy chain to the human α-L-iduronidase with the amino acid sequence of SEQ ID NO: 20 via the linker of the amino acid sequence of SEQ ID NO: 3, thereby to form an amino acid sequence of SEQ ID NO: 24.

28. The pharmaceutical composition according to any one of claims 1 to 27, wherein the pharmaceutical composition is a lyophilized preparation or an aqueous liquid preparation.

29. The pharmaceutical composition according to claim 28, further comprising at least one of a neutral salt, a disaccharide, a nonionic surfactant, and a buffer.

30. The pharmaceutical composition according to claim 28 or 29, wherein the pharmaceutical composition comprises a polysorbate and/or a poloxamer as the nonionic surfactant.

31. The pharmaceutical composition according to claim 30, wherein
the polysorbate is polysorbate 20 or polysorbate 80, and
the poloxamer is selected from the group consisting of poly(oxyethylene) (54) poly(oxypropylene) (39) glycol, poly(oxyethylene) (196) poly(oxypropylene) (67) glycol, poly(oxyethylene) (42) poly(oxypropylene) (67) glycol, poly(oxyethylene) (3) poly(oxypropylene) (17) glycol, poly(oxyethylene) (20) poly(oxypropylene) (20) glycol, and poly(oxyethylene) (120) poly(oxypropylene) (40) glycol.

32. The pharmaceutical composition according to claim 30, wherein
the polysorbate is polysorbate 80, and
the poloxamer is poly(oxyethylene) (160) poly(oxypropylene) (30) glycol.

33. The pharmaceutical composition according to any one of claims 30 to 32, wherein
the pharmaceutical composition is an aqueous liquid preparation,
a concentration of the polysorbate is from 0.005 to 1.5 mg/mL, and
a concentration of the poloxamer is from 0.1 to 0.6 mg/mL.

34. The pharmaceutical composition according to any one of claims 30 to 32, wherein
the pharmaceutical composition is an aqueous liquid preparation,
a concentration of the polysorbate is from 0.025 to 1.0 mg/mL, and
a concentration of the poloxamer is from 0.2 to 0.5 mg/mL.

35. The pharmaceutical composition according to any one of claims 30 to 32, wherein
the pharmaceutical composition is an aqueous liquid preparation,
a concentration of the polysorbate is from 0.05 to 0.15 mg/mL, and
a concentration of the poloxamer is from 0.25 to 0.45 mg/mL.

36. The pharmaceutical composition according to any one of claims 29 to 35, wherein the neutral salt is sodium chloride.

37. The pharmaceutical composition according to any one of claims 29 to 36, wherein the disaccharide is selected from the group consisting of trehalose, sucrose, maltose, lactose, and a combination of two or more thereof.

38. The pharmaceutical composition according to any one of claims 29 to 37, wherein the buffer is selected from the group consisting of a citrate buffer, a phosphate buffer, a glycine buffer, a histidine buffer, a carbonate buffer, an acetate buffer, and a combination of two or more thereof.

39. The pharmaceutical composition according to claim 30 or 31, wherein the pharmaceutical composition is an aqueous liquid preparation selected from the group consisting of:
(1) an aqueous liquid preparation with a concentration of the fusion protein of 1 to 10 mg/mL, a concentration of the neutral salt of 0.3 to 1.2 mg/mL, a concentration of the disaccharide of 50 to 100 mg/mL, a concentration of the buffer of 10 to 30 mM, a concentration of the polysorbate of 0.005 to 1.5 mg/mL, and a concentration of the poloxamer of 0.1 to 0.6 mg/mL;
(2) an aqueous liquid preparation with a concentration of the fusion protein of 2 to 8 mg/mL, a concentration of the neutral salt of 0.5 to 1.0 mg/mL, a concentration of the disaccharide of 55 to 95 mg/mL, a concentration of the buffer of 15 to 25 mM, a concentration of the polysorbate of 0.05 to 1.0 mg/mL, and a concentration of the poloxamer of 0.25 to 0.45 mg/mL; and
(3) an aqueous liquid preparation with a concentration of the fusion protein of 4 to 6 mg/mL, a concentration of the neutral salt of 0.7 to 0.9 mg/mL, a concentration of the disaccharide of 60 to 90 mg/mL, a concentration of the buffer of 15 to 25 mM, a concentration of the polysorbate of 0.05 to 0.15 mg/mL, and a concentration of the poloxamer of 0.25 to 0.45 mg/mL.

40. The pharmaceutical composition according to any one of claims 28 to 41, wherein the pharmaceutical composition is an aqueous liquid preparation with a pH of 4.5 to 6.5, 5.0 to 6.0, or 5.2 to 5.8.

41. The pharmaceutical composition according to any one of claims 30 to 32, wherein the pharmaceutical composition is a lyophilized preparation selected from the group consisting of:
(1) a lyophilized preparation giving a concentration of the fusion protein of 1 to 10 mg/mL, a concentration of the neutral salt of 0.3 to 1.2 mg/mL, a concentration of the disaccharide of 50 to 100 mg/mL, a concentration of the buffer of 10 to 30 mM, a concentration of the polysorbate of 0.005 to 1.5 mg/mL, and a concentration of the poloxamer of 0.1 to 0.6 mg/mL when dissolved in pure water;
(2) a lyophilized preparation giving a concentration of the fusion protein of 2 to 8 mg/mL, a concentration of the neutral salt of 0.5 to 1.0 mg/mL, a concentration of the disaccharide of 55 to 95 mg/mL, a concentration of the buffer of 15 to 25 mM, a concentration of the polysorbate of 0.05 to 1.0 mg/mL, and a concentration of the poloxamer of 0.25 to 0.45 mg/mL when dissolved in pure water; and
(3) a lyophilized preparation giving a concentration of the fusion protein of 4 to 6 mg/mL, a concentration of the neutral salt of 0.7 to 0.9 mg/mL, a concentration of the disaccharide of 60 to 90 mg/mL, a concentration of the buffer of 15 to 25 mM, a concentration of the polysorbate of 0.05 to 0.15 mg/mL, and a concentration of the poloxamer of 0.25 to 0.45 mg/mL when dissolved in pure water.

42. The pharmaceutical composition according to any one of claims 28 to 32, 36 to 38, and 41, wherein the pharmaceutical composition is a lyophilized preparation giving a pH of 4.5 to 6.5, 5.0 to 6.0, or 5.2 to 5.8 when dissolved in pure water.

43. The pharmaceutical composition according to any one of claims 1 to 42, wherein the patient has a disorder in the central nervous system.

44. The pharmaceutical composition according to any one of claims 1 to 43, wherein the pharmaceutical composition has an effect of reducing concentrations of dermatan sulfate and heparan sulfate contained in cerebrospinal fluid, serum, and urine.

45. The pharmaceutical composition according to any one of claims 1 to 44, wherein the pharmaceutical composition is used in enzyme replacement therapy for a patient with mucopolysaccharidosis type I.

46. The pharmaceutical composition according to any one of claims 1 to 45, wherein the pharmaceutical composition is used in combination with an immunosuppressive agent.

47. Enzyme replacement therapy for a patient with mucopolysaccharidosis type I, the enzyme replacement therapy using the pharmaceutical composition described in any of claims 1 to 46.

48. The enzyme replacement therapy according to claim 47, wherein the patient has a disorder in the central nervous system.
